# FASCICULE DE BREVET EUROPEEN

(11) **EP 2 338 572 B1**
(45) Date de publication et mention de la délivrance du brevet: **23.05.2012**
(21) Numéro de dépôt: 10194754.7
(22) Date de dépôt: 13.12.2010
(51) Int. Cl.: A61Q 5/08, A61Q 5/10, A61K 8/22, A61K 8/31, A61K 8/41, A61K 8/44

(54) **Agent de coloration et/ou de décoloration des fibres kératiniques en deux parties, comprenant un corps gras et un agent séquestrant**
Substanz zum Färben und/oder Entfärben von Keratinfasern bestehend aus zwei Komponenten, die einen Fettkörper und ein Sequestriermittel enthält
Agent for colouring and/or bleaching keratinous fibres in two parts including a fatty body and a sequestering agent

(30) Priorité: 22.12.2009 FR 0959433
(43) Date de publication de la demande: 29.06.2011
(73) Titulaire: L'Oréal, 75008 Paris (FR)
(72) Inventeur: Deconinck, Gautier, 95210, Saint Gratien (FR); Goget, Caroline, 75018, Paris (FR)
(74) Mandataire: Dossmann, Gérard

(56) Documents cités:
- EP-A1- 2 198 843
- EP-A1- 2 198 849
- EP-A2- 2 198 842
- FR-A1- 2 925 308
- FR-A1- 2 940 054

## Description

La présente invention concerne un agent en deux parties, destiné à la coloration et/ou la décoloration des fibres kératiniques, en particulier des fibres kératiniques humaines telles que les cheveux.

Plus précisément, la présente invention a pour objet un agent de coloration et/ou de décoloration des fibres kératiniques, constitué d'une première composition (A) contenant un ou plusieurs agents alcalinisants et éventuellement un ou plusieurs colorants, et d'une seconde composition (B) contenant un ou plusieurs agents oxydants, l'une au moins des deux compositions (A) et (B) comprenant un ou plusieurs corps gras ne contenant pas de fonction acide carboxylique, et un ou plusieurs agents séquestrants particuliers.

La présente invention concerne également un dispositif à plusieurs compartiments, contenant l'agent de coloration et/ou de décoloration selon l'invention.

La présente invention a enfin pour objet un procédé de coloration et/ou de décoloration des fibres kératiniques, mettant en oeuvre l'agent selon l'invention.

Depuis longtemps, de nombreuses personnes cherchent à modifier la couleur de leurs cheveux, et notamment à les décolorer, ou au contraire à les colorer par exemple afin de masquer leurs cheveux blancs.

Pour colorer les fibres kératiniques humaines, essentiellement deux types de colorations ont été développés.

Le premier type de coloration est la coloration dite permanente ou d'oxydation, qui met en oeuvre des compositions tinctoriales contenant des précurseurs de colorant d'oxydation, appelés généralement bases d'oxydation. Ces bases d'oxydation sont des composés incolores ou faiblement colorés qui, associés à des produits oxydants, peuvent donner naissance par un processus de condensation oxydative à des composés colorés.

On sait également que l'on peut faire varier les nuances obtenues avec ces bases d'oxydation en les associant à des coupleurs ou modificateurs de coloration. La variété des molécules mises en jeu au niveau des bases d'oxydation et des coupleurs permet l'obtention d'une riche palette de couleurs.

Le deuxième type de coloration est la coloration dite semi-permanente ou coloration directe, qui consiste à appliquer sur les fibres kératiniques des colorants directs qui sont des molécules colorées et colorantes ayant une affinité pour lesdites fibres, à laisser pauser, puis à les rincer.

Afin de réaliser ces colorations, les colorants directs généralement employés sont choisis parmi les colorants directs nitrés benzéniques, anthraquinoniques, nitropyridiniques, azoïques, xanthéniques, acridiniques, aziniques ou triarylméthaniques.

Ce type de procédé ne nécessite pas l'emploi d'un agent oxydant pour développer la coloration. Cependant, il est possible de mettre en oeuvre un tel agent, afin d'obtenir un effet d'éclaircissement avec la coloration. On parle alors d'une coloration directe ou semi-permanente en conditions éclaircissantes.

Les procédés de coloration permanente ou semi-permanente en conditions éclaircissantes nécessitent donc d'employer, avec la composition tinctoriale, une composition aqueuse comprenant au moins un agent oxydant, en condition de pH alcalin dans la grande majorité des cas.

Les procédés classiques de décoloration des fibres kératiniques humaines consistent à mettre en oeuvre une composition aqueuse comprenant au moins un agent oxydant dans des conditions de pH généralement alcalin. Cet agent oxydant a pour rôle de dégrader la mélanine des cheveux, ce qui, en fonction de la nature de l'agent oxydant présent, conduit à un éclaircissement plus ou moins prononcé des fibres. Ainsi, pour un éclaircissement relativement faible, l'agent oxydant est généralement le peroxyde d'hydrogène. Lorsqu'un éclaircissement plus important est recherché, l'on met habituellement en oeuvre des sels peroxygénés, tels que par exemple des persulfates, en présence de peroxyde d'hydrogène.

L'une des difficultés rencontrées lors de la mise en oeuvre des procédés de coloration et de décoloration de l'art antérieur vient du fait qu'ils sont mis en oeuvre dans des conditions alcalines.

Afin d'améliorer les performances des procédés de coloration et/ou de décoloration des fibres kératiniques humaines, et de limiter les désagréments liés à l'emploi d'agents alcalins et d'agents oxydants, il a été proposé d'employer dans les compositions tinctoriales une quantité substantielle d'un ou plusieurs corps gras.

Cependant, lors du mélange, enrichi en corps gras, d'une composition comprenant un agent alcalin et d'une composition comprenant un agent oxydant, il se produit un gonflement du mélange lié à un dégagement d'oxygène.

Ce phénomène est d'autant plus gênant lorsque le mélange des composants est réalisé juste avant l'application sur les fibres : le gonflement progressif du mélange au cours du temps gène son application sur les fibres, et rend moins précise cette application. Ceci peut engendrer également une mauvaise homogénéité de la coloration et/ou de la décoloration.

La Demanderesse a maintenant découvert que l'emploi de certains agents séquestrants particuliers permet de réduire ce phénomène de gonflement, et d'obtenir un mélange qui évolue peu au cours du temps, y compris lors de son application sur les fibres kératiniques.

La présente invention a donc pour objet un agent de coloration et/ou de décoloration des fibres kératiniques, constitué par :
- une première composition (A) comprenant un ou plusieurs agents alcalinisants, et
- une seconde composition (B) comprenant un ou plusieurs agents oxydants,
l'une au moins des deux compositions (A) et (B) comprenant un ou plusieurs corps gras ne contenant pas de fonction acide carboxylique, la quantité totale desdits corps gras dans le mélange des compositions (A) et (B) représentant au moins 20% en poids, par rapport au poids total dudit mélange, et
l'une au moins des deux compositions (A) et (B) comprenant un ou plusieurs agents séquestrants de formule (I) suivante : 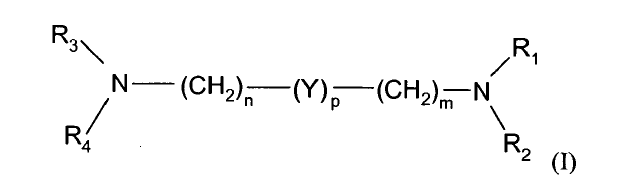 dans laquelle :
p est un nombre entier valant 0 ou 1,
n et m sont des nombres entiers valant, indépendamment l'un de l'autre, 0, 1 ou 2, la somme n + m valant au moins 1,
R₁, R₂, R₃ et R₄ représentent, indépendamment les uns des autres, un atome d'hydrogène, un groupe -CH₂CO₂M1, ou un groupe -CH(CO₂M2)(CH₂CO₂M3),
Y représente un groupe NCH₂CO₂M4,
M1 à M4 désignant, indépendamment les uns des autres, un atome d'hydrogène, un cation issu d'un métal alcalin ou d'un métal alcalino-terreux, un cation issu d'une amine organique éventuellement hydroxylée, ou un cation ammonium,
à la condition que, si p vaut 0, alors R₁ et R₃ représentent tous deux un atome d'hydrogène, et R₂ et R₄ représentent tous deux un groupe -CH(CO₂M2)(CH₂CO₂M3), et
si p vaut 1, alors n et m valent chacun au moins 1.

Lorsque l'agent selon l'invention est destiné à la coloration, des fibres kératiniques, la composition (A) comprend en outre un ou plusieurs colorants d'oxydation et/ou un ou plusieurs colorants directs.

A l'inverse, lorsque l'agent selon l'invention est destiné à la seule décoloration des fibres kératiniques, les compositions (A) et (B) ne comprennent pas de colorant direct ni de colorant d'oxydation (bases et coupleurs) ou bien, s'ils sont présents, leur teneur totale ne dépasse pas 0,005% en poids par rapport au poids de chaque composition. En effet, à une telle teneur, seule la composition serait teintée, c'est-à-dire qu'on n'observerait pas d'effet de coloration des fibres kératiniques.

L'agent de coloration et/ou de décoloration selon la présente invention n'évolue pas ou peu au cours du temps lors du mélange des deux compositions (A) et (B), ou de l'application séquentielle de ces deux compositions sur les fibres kératiniques. Il présente ainsi une facilité d'application et une très bonne efficacité, notamment en termes de qualité et d'homogénéité de la coloration et/ou de la décoloration.

En outre, lorsqu'il est destiné à la coloration, l'agent selon l'invention est particulièrement efficace sur le plan de la puissance de la coloration obtenue, ainsi que sur celui de la chromaticité, et de la sélectivité de la coloration d'une même fibre, ou entre des fibres différemment sensibilisées.

Lorsqu'il est destiné à la décoloration, l'agent selon l'invention présente des performances éclaircissantes équivalentes voire supérieures à celles obtenues avec les compositions existantes, notamment avec celles à base d'hydroxyde d'ammonium.

L'agent selon l'invention présente également l'avantage de limiter les odeurs agressives lors de sa préparation, ou de son application sur les fibres.

D'autres caractéristiques et avantages de l'invention apparaîtront plus clairement à la lecture de la description et des exemples qui suivent.

Dans ce qui va suivre, et à moins d'une autre indication, les bornes d'un domaine de valeurs sont comprises dans ce domaine.

Les fibres kératiniques humaines traitées par le procédé selon l'invention sont de préférence les cheveux.

Selon la présente invention, la composition (A) comprend un ou plusieurs agents alcalinisants.

Par agent alcalinisant on entend au sens de l'invention tout composé qui, par sa présence dans la composition (A), augmente le pH de cette composition d'au moins 0,05 unité de pH et de préférence d'au moins 0,1 unité de pH.

L'agent alcalinisant peut être en particulier une base minérale ou organique.

De préférence, l'agent alcalinisant est choisi parmi l'ammoniaque, les carbonates alcalins, les alcanolamines ainsi que leurs dérivés, les hydroxydes de sodium ou de potassium, et les composés de formule (II) suivante : 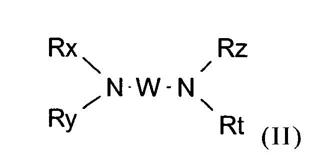 dans laquelle W est un reste alkylène en C₁-C₆ éventuellement substitué par un groupement hydroxyle ou un radical alkyle en C₁-C₆; Rx, Ry, Rz et Rt, identiques ou différents, représentent un atome d'hydrogène, un radical alkyle en C₁-C₆, hydroxyalkyle en C₁-C₆, ou aminoalkyle en C₁-C₆.

On peut citer à titre d'exemple de tels composés de formule (II) le 1,3 diaminopropane, le 1,3 diamino 2 propanol, la spermine, la spermidine.

Les agents alcalinisants particulièrement préférés sont les alcanolamines, et en particulier les mono-, di- et triéthanolamines.

Dans une variante préférée de l'invention, l'agent alcalinisant est la monoéthanolamine.

Selon un mode de réalisation particulier, la composition (A) contient en tant qu'agent alcalinisant au moins une amine organique, de préférence au moins une alcanolamine. Lorsque la composition (A) contient plusieurs agents alcalinisants dont une alcanolamine et de l'ammoniaque ou un de ses sels, la ou les amines organiques sont de préférence majoritaires en poids, par rapport à la quantité d'ammoniac présent dans la composition (A).

Selon un mode de réalisation préféré de la présente invention, la composition (A) ne contient pas d'ammoniaque.

Selon un mode de réalisation également préféré de la présente invention, lorsque la composition (A) contient de l'ammoniaque ou un de ses sels, elle contient également une ou plusieurs alcanolamines, et la quantité pondérale d'alcanolamine(s) dans la composition (A) est supérieure à la quantité pondérale d'ammoniac dans cette même composition.

Généralement, la composition (A) présente une teneur en agent(s) alcalinisant(s) allant de 0,1 à 40 % en poids, de préférence de 0,5 à 20 % en poids, par rapport au poids de cette composition.

De préférence, la composition (A) présente un pH supérieur ou égal à 8, et plus préférentiellement un pH allant de 8,5 à 11,5.

Ce pH peut également être ajusté à la valeur souhaitée par l'emploi, en plus de l'agent alcalinisant, d'un ou plusieurs agents acidifiants.

Parmi les agents acidifiants, on peut citer à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Selon la présente invention, la composition (B) comprend un ou plusieurs agents oxydants,

Cet agent oxydant peut être choisi parmi les agents oxydants classiquement utilisés pour la décoloration et la teinture d'oxydation des fibres kératiniques, et parmi lesquels on peut citer le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux comme le sodium, le potassium, le magnésium. On peut également utiliser à titre d'agent oxydant une ou plusieurs enzymes d'oxydo-réduction telles que les laccases, les peroxydases et les oxydoréductases à 2 électrons (telles que l'uricase), éventuellement en présence de leur donneur ou cofacteur respectif.

L'utilisation du peroxyde d'hydrogène est particulièrement préférée. Celui-ci peut être avantageusement employé en solution aqueuse (eau oxygénée) dont la concentration peut varier, plus particulièrement de 0,1 1 à 50% en poids, et encore plus préférentiellement de 0,5 à 20% en poids, mieux de 1 à 15% en poids par rapport au poids total de la composition (B).

En fonction du degré de décoloration souhaité, l'agent oxydant peut également comprendre un ou plusieurs composés choisis de préférence parmi les sels peroxygénés.

De préférence, le pH de la composition (B) est inférieur à 7. Ce pH peut être ajusté à la valeur souhaitée par l'emploi d'un ou plusieurs agents acidifiants, qui peuvent être notamment choisis parmi ceux décrits précédemment.

Selon la présente invention, l'une et/ou l'autre des deux compositions (A) et (B) comprennent un ou plusieurs agents séquestrants de formule (I) telle que définie ci-avant.

Dans la formule (I) ci-avant, M1 à M4 désignent, indépendamment les uns des autres, un atome d'hydrogène, un cation issu d'un métal alcalin ou d'un métal alcalino-terreux, un cation issu d'une amine organique éventuellement hydroxylée, ou un cation ammonium.

A titre d'exemples de cations de métaux alcalins, on peut notamment citer le sodium (Na⁺) et le potassium (K⁺) ; à titre d'exemples de cations de métaux alcalino-terreux, on peut notamment citer le calcium (Ca²⁺) et le magnésium (Mg²⁺).

En ce qui concerne les cations d'amines organiques, on peut citer les cations d'amines primaires, secondaires ou tertiaires, ou encore d'alcanolamines.

Lesdites amines présentent un ou plusieurs radicaux, identiques ou non, de type alkyle ou hydroxyalkyle, linéaire ou ramifié en C₁ à C₂₀.

De préférence, M1 à M4 sont identiques, et désignent un cation sodium ou un cation potassium.

Selon la présente invention, les agents séquestrants particulièrement préférés sont :
- l'acide diethylènetriamine pentaacétique (DTPA) et ses sels,
- l'acide éthylènediamine disuccinique (EDDS) et ses sels.

Parmi les sels de ces deux composés, on préfère les sels de métaux alcalins, et notamment les sels de sodium ou de potassium.

Selon la présente invention, le ou les agents séquestrants peuvent être présents dans la composition (A), dans la composition (B), ou dans ces deux compositions à la fois.

Selon un mode de réalisation préféré, la composition (A) comprend un ou plusieurs agents séquestrants tels que défini ci-avant.

Selon un mode de réalisation particulier, l'agent séquestrant est uniquement présent dans la composition (A).

Qu'il soit présent dans l'une, l'autre, ou les deux compositions (A) et (B), la quantité totale d'agent séquestrant de formule (I) dans le mélange de ces deux compositions est avantageusement de 0,001 à 10% en poids, de préférence de 0,01 à 5% en poids, et mieux encore de 0,05 à 1% en poids, par rapport au poids total du mélange des deux compositions (A) et (B). Ces pourcentages en poids sont exprimés par rapport à la forme acide du ou des composés de formule (I).

Ainsi que cela a été mentionné, l'une au moins des deux compositions (A) et (B) comprend un ou plusieurs corps gras ne contenant pas de fonction acide carboxylique, la quantité totale de tels corps gras dans le mélange des compositions (A) et (B) représentant au moins 20% en poids, par rapport au poids total du mélange de ces deux compositions.

Ainsi, le ou les corps gras ne contenant pas de fonction acide carboxylique peuvent être présents dans la composition (A), ou la composition (B), ou les deux compositions à la fois, pourvu que la quantité totale de ces corps gras dans le mélange des compositions (A) et (B) soit au moins égale à 20% en poids, par rapport au poids total du mélange des deux compositions (A) et (B).

De préférence, la quantité totale de corps gras ne contenant pas de fonction acide carboxylique dans le mélange des compositions (A) et (B) représente au moins 25% en poids, et plus préférentiellement au moins 30% en poids, par rapport au poids total dudit mélange.

La quantité totale de corps gras ne contenant pas de fonction acide carboxylique dans le mélange des compositions (A) et (B) est avantageusement inférieure ou égale à 90% en poids, et de préférence inférieure ou égale à 70% en poids, par rapport au poids total dudit mélange.

Selon un mode de réalisation préféré, la composition (A) comprend un ou plusieurs corps gras ne contenant pas de fonction acide carboxylique.

Par corps gras, on entend dans la présente invention, un composé organique insoluble dans l'eau à température ordinaire (25°C) et à pression atmosphérique (760 mm de Hg), c'est-à-dire qui présente une solubilité en poids dans l'eau inférieure à 5% et de préférence à 1 % encore plus préférentiellement à 0,1%. Les corps gras présentent dans leur structure au moins un enchaînement d'au moins deux groupements siloxanes ou une chaîne hydrocarbonée comportant au moins 6 atomes de carbone. En outre, les corps gras sont généralement solubles dans les solvants organiques dans les mêmes conditions de température et de pression, comme par exemple le chloroforme, l'éthanol, le benzène, l'huile de vaseline ou le décaméthylcyclopentasiloxane.

Dans le cadre de l'invention, les corps gras contenant une ou plusieurs fonctions acide carboxylique terminale tels que les acides gras sont exclus, qui présentent une certaine solubilité dans l'eau notamment en milieu alcalin. Par corps gras ne contenant pas de fonction acide carboxylique, on désigne un corps gras ne contenant pas de groupement -COOH, ni de groupement -COO .

Selon l'invention, les corps gras ne contenant pas de fonction acide carboxylique sont choisis parmi les composés liquides ou pâteux à température ambiante et à pression atmosphérique, et de préférence les composés liquides.

Le ou les corps gras ne contenant pas de fonction acide carboxylique sont notamment choisis parmi les alcanes inférieurs en C₆-C₁₆, les huiles non siliconées d'origine minérale, végétale, animale ou synthétique, les alcools gras, les esters d'acide gras, les esters d'alcool gras, les cires non siliconées et les silicones.

Il est précisé qu'au sens de l'invention, les alcools gras et acides gras présentent plus particulièrement un ou plusieurs groupements hydrocarbonés, linéaires ou ramifiés, saturés ou insaturés, comprenant 6 à 30 atomes de carbone, éventuellement substitués, en particulier par un ou plusieurs groupements hydroxyle (en particulier 1 à 4). S'ils sont insaturés, ces composés peuvent comprendre une à trois double-liaisons carbone-carbone, conjuguées ou non.

En ce qui concerne les alcanes inférieurs en C₆-C₁₆, ces derniers sont linéaires ou ramifiés, éventuellement cycliques. A titre d'exemple, on peut citer l'hexane et le dodécane, les isoparaffines comme l'isohexadécane et l'isodécane.

Comme huiles d'origine minérale, végétale, animale ou synthétique utilisables dans la présente invention, on peut citer par exemple :
- les huiles hydrocarbonées d'origine animale, telles que le perhydrosqualène ;
- les huiles triglycérides d'origine végétale ou synthétique, telles que les triglycérides liquides d'acides gras comportant de 6 à 30 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol^{®} 810, 812 et 818 par la société Dynamit Nobel, l'huile de jojoba, l'huile de beurre de karité ;
- les hydrocarbures linéaires ou ramifiés de plus de 16 atomes de carbone, d'origine minérale ou synthétique, tels que les huiles de paraffine, la vaseline, l'huile de vaseline, les polydécènes, les polyisobutènes hydrogénés tels que Parléam^{®}.
- les huiles fluorées comme le perfluorométhylcyclopentane et le perfluoro-1,3 diméthylcyclohexane, vendus sous les dénominations de "FLUTEC^{®} PC1" et "FLUTEC^{®} PC3" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050^{®}" et "PF 5060^{®}" par la Société 3M, ou encore le bromoperfluorooctyle vendu sous la dénomination "FORALKYL^{®}" par la Société Atochem ; le nonafluoro-méthoxybutane et le nonafluoroéthoxyisobutane ; les dérivés de perfluoromorpholine, tels que la 4-trifluorométhyl perfluoromorpholine vendue sous la dénomination "PF 5052^{®}" par la Société 3M.

Les alcools gras convenant à la mise en oeuvre de l'invention sont plus particulièrement choisis parmi les alcools saturés ou insaturés, linéaires ou ramifiés, comportant de 8 à 30 atomes de carbone. On peut citer par exemple l'alcool cétylique, l'alcool l stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

En ce qui concerne les esters d'acides gras et/ou d'alcools gras, ils sont avantageusement différents des triglycérides mentionnés ci-dessus ; on peut citer notamment les esters de mono ou polyacides aliphatiques saturés ou insaturés, linéaires ou ramifiés en C₁-C₂₆ et de mono ou polyalcools aliphatiques saturés ou insaturés, linéaires ou ramifiés en C₁-C₂₆, le nombre total d'atomes de carbone des esters étant plus particulièrement supérieur ou égal à 10.

Parmi les monoesters, on peut citer le béhénate de dihydroabiétyle ; le béhénate d'octyldodécyle ; le béhénate d'isocétyle ; le lactate de cétyle ; le lactate d'alkyle en C₁₂-C₁₅ ; le lactate d'isostéaryle ; le lactate de lauryle ; le lactate de linoléyle ; le lactate d'oléyle ; l'octanoate de (iso)stéaryle ; l'octanoate d'isocétyle ; l'octanoate d'octyle ; l'octanoate de cétyle ; l'oléate de décyle ; l'isostéarate d'isocétyle ; le laurate d'isocétyle ; le stéarate d'isocétyle; l'octanoate d'isodécyle ; l'oléate d'isodécyle ; l'isononanoate d'isononyle ; le palmitate d'isostéaryle ; le ricinoléate de méthyle acétyle ; le stéarate de myristyle ; l'isononanoate d'octyle ; l'isononate de 2-éthylhexyle ; le palmitate d'octyle ; le pélargonate d'octyle ; le stéarate d'octyle ; l'érucate d'octyldodécyle ; l'érucate d'oléyle ; les palmitates d'éthyle et d'isopropyle, le palmitate d'éthyl-2-hexyle, le palmitate de 2-octyldécyle, les myristates d'alkyles tels que le myristate d'isopropyle, de butyle, de cétyle, de 2-octyldodécyle, de myristyle, de stéaryle le stéarate d'hexyle, le stéarate de butyle, le stéarate d'isobutyle ; le maléate de dioctyle, le laurate d'hexyle, le laurate de 2-hexyldécyle.

Toujours dans le cadre de cette variante, on peut également utiliser les esters d'acides di ou tricarboxyliques en C₄-C₂₂ et d'alcools en C₁-C₂₂ et les esters d'acides mono di ou tricarboxyliques et d'alcools di, tri, tétra ou pentahydroxy en C₂-C₂₆.

On peut notamment citer : le sébacate de diéthyle ; le sébacate de diisopropyle ; l'adipate de diisopropyle ; l'adipate de di n-propyle ; l'adipate de dioctyle ; l'adipate de diisostéaryle ; le maléate de dioctyle ; l'undecylénate de glycéryle ; le stéarate d'octyldodécyl stéaroyl ; le monoricinoléate de pentaérythrityle ; le tétraisononanoate de pentaérythrityle ; le tétrapélargonate de pentaérythrityle ; le tétraisostéarate de pentaérythrityle ; le tétraoctanoate de pentaérythrityle ; le dicaprylate de propylène glycol ; le dicaprate de propylène glycol, l'érucate de tridécyle ; le citrate de triisopropyle ; le citrate de triisotéaryle ; le trilactate de glycéryle ; le trioctanoate de glycéryle ; le citrate de trioctyldodécyle ; le citrate de trioléyle, le dioctanoate de propylène glycol ; le diheptanoate de néopentyl glycol ; le diisanonate de diéthylène glycol ; et les distéarates de polyéthylène glycol.

Parmi les esters cités ci-dessus, on préfère utiliser les palmitates d'éthyle, d'isopropyle, de myristyle, de cétyle, de stéaryle, le palmitate d'éthyl-2-hexyle, le palmitate de 2-octyldécyle, les myristates d'alkyles tels que le myristate d'isopropyle, de butyle, de cétyle, de 2-octyldodécyle, le stéarate d'hexyle, le stéarate de butyle, le stéarate d'isobutyle ; le maléate de dioctyle, le laurate d'hexyle, le laurate de 2-hexyldécyle et l'isononanate d'isononyle, l'octanoate de cétyle.

La composition peut également comprendre, à titre d'ester gras, des esters et di-esters de sucres d'acides gras en C₆-C₃₀, de préférence en C₁₂-C₂₂. Il est rappelé que l'on entend par « sucre », des composés hydrocarbonés oxygénés qui possèdent plusieurs fonctions alcool, avec ou sans fonction aldéhyde ou cétone, et qui comportent au moins 4 atomes de carbone. Ces sucres peuvent être des monosaccharides, des oligosaccharides ou des polysaccharides.

Comme sucres convenables, on peut citer par exemple le sucrose (ou saccharose), le glucose, le galactose, le ribose, le fucose, le maltose, le fructose, le mannose, l'arabinose, le xylose, le lactose, et leurs dérivés notamment alkylés, tels que les dérivés méthylés comme le méthylglucose.

Les esters de sucres et d'acides gras peuvent être choisis notamment dans le groupe comprenant les esters ou mélanges d'esters de sucres décrits auparavant et d'acides gras en C₆-C₃₀, de préférence en C₁₂-C₂₂, linéaires ou ramifiés, saturés ou insaturés. S'ils sont insaturés, ces composés peuvent comprendre une à trois double-liaisons carbone-carbone, conjuguées ou non.

Les esters selon cette variante peuvent être également choisis parmi les mono-, di-, tri- et tétra-esters, les polyesters et leurs mélanges.

Ces esters peuvent être par exemple des oléate, laurate, palmitate, myristate, béhénate, cocoate, stéarate, linoléate, linolénate, caprate, arachidonates, ou leurs mélanges comme notamment les esters mixtes oléo-palmitate, oléo-stéarate, palmito-stéarate.

Plus particulièrement, on utilise les mono- et di- esters et notamment les mono- ou di- oléate, stéarate, béhénate, oléopalmitate, linoléate, linolénate, oléostéarate, de saccharose, de glucose ou de méthylglucose.

On peut citer à titre d'exemple le produit vendu sous la dénomination Glucate® DO par la société Amerchol, qui est un dioléate de méthylglucose.

On peut aussi citer à titre d'exemples d'esters ou de mélanges d'esters de sucre d'acide gras :
- les produits vendus sous les dénominations F160, F140, F110, F90, F70, SL40 par la société Crodesta, désignant respectivement les palmito-stéarates de sucrose formés de 73 % de monoester et 27 % de di- et tri-ester, de 61 % de monoester et 39 % de di-, tri-, et tétra-ester, de 52 % de monoester et 48 % de di-, tri-, et tétra-ester, de 45 % de monoester et 55 % de di-, tri-, et tétra-ester, de 39 % de monoester et 61 % de di-, tri-, et tétra-ester, et le mono-laurate de sucrose;
- les produits vendus sous la dénomination Ryoto Sugar Esters par exemple référencés B370 et correspondant au béhénate de saccharose formé de 20 % de monoester et 80 % de di-triester-polyester;
- le mono-di-palmito-stéarate de sucrose commercialisé par la société Goldschmidt sous la dénomination Tegosoft® PSE.

La cire ou les cires non siliconées susceptibles d'être utilisées comme corps gras sont choisies notamment, parmi la cire de Carnauba, la cire de Candelila, et la cire d'Alfa, la cire de paraffine, l'ozokérite, les cires végétales comme la cire d'olivier, la cire de riz, la cire de jojoba hydrogénée ou les cires absolues de fleurs telles que la cire essentielle de fleur de cassis vendue par la société BERTIN (France), les cires animales comme les cires d'abeilles, ou les cires d'abeilles modifiées (cerabellina) ; d'autres cires ou matières premières cireuses utilisables selon l'invention sont notamment les cires marines telles que celle vendue par la Société SOPHIM sous la référence M82, les cires de polyéthylène ou de polyoléfines en général.

Les silicones utilisables comme corps gras sont des silicones volatiles ou non volatiles, cycliques, linéaires ou ramifiées, modifiées ou non par des groupements organiques, ayant une viscosité de 5.10⁻⁶ à 2,5m²/s à 25°C et de préférence 1.10⁻⁵ à 1m²/s.

Les silicones utilisables conformément à l'invention peuvent se présenter sous forme d'huiles, de cires, de résines ou de gommes.

De préférence, la silicone est choisie parmi les polydialkylsiloxanes, notamment les polydiméthylsiloxanes (PDMS), et les polysiloxanes organo-modifiés comportant au moins un groupement fonctionnel choisi parmi les groupements poly(oxyalkylène), les groupements aminés et les groupements alcoxy.

Les organopolysiloxanes sont définis plus en détail dans l'ouvrage de Walter NOLL "Chemistry and Technology of Silicones" (1968), Academie Press. Elles peuvent être volatiles ou non volatiles.

Lorsqu'elles sont volatiles, les silicones sont plus particulièrement choisies parmi celles possédant un point d'ébullition compris entre 60°C et 260°C, et plus particulièrement encore parmi:
(i) les polydialkylsiloxanes cycliques comportant de 3 à 7, de préférence de 4 à 5 atomes de silicium. Il s'agit, par exemple, de l'octaméthylcyclotétrasiloxane commercialisé notamment sous le nom de VOLATILE SILICONE^{®} 7207 par UNION CARBIDE ou SILBIONE^{®} 70045 V2 par RHODIA, le décaméthylcyclopentasiloxane commercialisé sous le nom de VOLATILE SILICONE^{®} 7158 par UNION CARBIDE, et SILBIONE® 70045 V5 par RHODIA, ainsi que leurs mélanges.

On peut également citer les cyclocopolymères du type diméthylsiloxanes/ méthylalkylsiloxane, tel que la SILICONE VOLATILE^{®} FZ 3109 commercialisée par la société UNION CARBIDE, de formule :

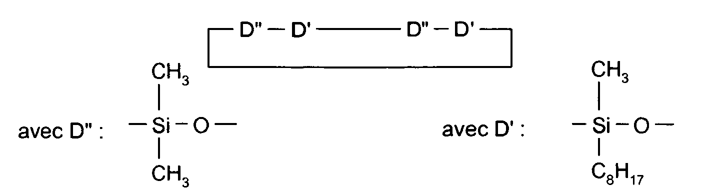

On peut également citer les mélanges de polydialkylsiloxanes cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1 ,1'-(hexa-2,2,2',2',3,3'-triméthylsilyloxy) bis-néopentane ;
(ii) les polydialkylsiloxanes volatiles linéaires ayant 2 à 9 atomes de silicium et présentant une viscosité inférieure ou égale à 5.10⁻⁶ m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and Toiletries, Vol. 91, Jan. 76, P. 27-32 - TODD & BYERS "Volatile Silicone fluids for cosmetics".

On utilise de préférence des polydialkylsiloxanes non volatiles, des gommes et des résines de polydialkylsiloxanes, des polyorganosiloxanes modifiés par les groupements organofonctionnels ci-dessus ainsi que leurs mélanges.

Ces silicones sont plus particulièrement choisies parmi les polydialkylsiloxanes parmi lesquels on peut citer principalement les polydiméthylsiloxanes à groupements terminaux triméthylsilyl. La viscosité des silicones est mesurée à 25°C selon la norme ASTM 445 Appendice C.

Parmi ces polydialkylsiloxanes, on peut citer à titre non limitatif les produits commerciaux suivants :
- les huiles SILBIONE® des séries 47 et 70 047 ou les huiles MIRASIL® commercialisées par RHODIA telles que, par exemple l'huile 70 047 V 500 000 ;
- les huiles de la série MIRASIL® commercialisées par la société RHODIA ;
- les huiles de la série 200 de la société DOW CORNING telles que la DC200 ayant viscosité 60 000 mm²/s ;
- les huiles VISCASIL® de GENERAL ELECTRIC et certaines huiles des séries SF (SF 96, SF 18) de GENERAL ELECTRIC.

On peut également citer les polydiméthylsiloxanes à groupements terminaux diméthylsilanol connus sous le nom de diméthiconol (CTFA), tels que les huiles de la série 48 de la société RHODIA.

Dans cette classe de polydialkylsiloxanes, on peut également citer les produits commercialisés sous les dénominations "ABIL WAX® 9800 et 9801" par la société GOLDSCHMIDT qui sont des polydialkyl (C₁-C₂₀) siloxanes.

Les gommes de silicone utilisables conformément à l'invention sont notamment des polydialkylsiloxanes, de préférence des polydiméthylsiloxanes ayant des masses moléculaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles polyphénylméthylsiloxanes (PPMS), les isoparaffines, les polyisobutylènes, le chlorure de méthylène, le pentane, le dodécane, le tridécane ou leurs mélanges.

Des produits plus particulièrement utilisables conformément à l'invention sont des mélanges tels que :
- les mélanges formés à partir d'un polydiméthylsiloxane hydroxylé en bout de chaîne, ou diméthiconol (CTFA) et d'un polydiméthylsiloxane cyclique également appelé cyclométhicone (CTFA) tel que le produit Q2 1401 commercialisé par la société DOW CORNING ;
- les mélanges d'une gomme polydiméthylsiloxane et d'une silicone cyclique tel que le produit SF 1214 Silicone Fluid de la société GENERAL ELECTRIC, ce produit est une gomme SF 30 correspondant à une diméthicone, ayant un poids moléculaire moyen en nombre de 500 000 solubilisée dans l'huile SF 1202 Silicone Fluid correspondant au décaméthylcyclopentasiloxane ;
- les mélanges de deux PDMS de viscosités différentes, et plus particulièrement d'une gomme PDMS et d'une huile PDMS, tels que le produit SF 1236 de la société GENERAL ELECTRIC. Le produit SF 1236 est le mélange d'une gomme SE 30 définie ci-dessus ayant une viscosité de 20 m²/s et d'une huile SF 96 d'une viscosité de 5.10⁻⁶m²/s. Ce produit comporte de préférence 15 % de gomme SE 30 et 85 % d'une huile SF 96.

Les résines d'organopolysiloxanes utilisables conformément à l'invention sont des systèmes siloxaniques réticulés renfermant les motifs :
R₂SiO_{2/2}, R₃SiO_{1/2}, RoSiO_{3/2} et SiO_{4/2}
dans lesquelles R représente un alkyl possédant 1 à 16 atomes de carbone. Parmi ces produits, ceux particulièrement préférés sont ceux dans lesquels R désigne un groupe alkyle inférieur en C₁-C₄, plus particulièrement méthyle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Les silicones organomodifiées utilisables conformément à l'invention sont des silicones telles que définies précédemment et comportant dans leur structure un ou plusieurs groupements organofonctionnels fixés par l'intermédiaire d'un groupe hydrocarboné.

Outre les silicones décrites ci-dessus, les silicones organomodifiées peuvent être des polydiaryl siloxanes, notamment des polydiphénylsiloxanes, et des polyalkyl-arylsiloxanes fonctionnalisés par les groupes organofonctionnels mentionnés précédemment.

Les polyalkylarylsiloxanes sont particulièrement choisis parmi les polydiméthyl/méthylphénylsiloxanes, les polydiméthyl/diphénylsiloxanes linéaires et/ou ramifiés de viscosité allant de 1.10⁻⁵ à 5.10⁻²m²/s à 25°C.

Parmi ces polyalkylarylsiloxanes, on peut citer à titre d'exemple les produits commercialisés sous les dénominations suivantes :
- les huiles SILBIONE® de la série 70 641 de RHODIA;
- les huiles des séries RHODORSIL® 70 633 et 763 de RHODIA ;
- l'huile DOW CORNING 556 COSMETIC GRAD FLUID de DOW CORNING ;
- les silicones de la série PK de BAYER comme le produit PK20 ;
- les silicones des séries PN, PH de BAYER comme les produits PN1000 et PH1000 ;
- certaines huiles des séries SF de GENERAL ELECTRIC telles que SF 1023, SF 1154, SF 1250, SF 1265.

Parmi les silicones organomodifiées, on peut citer les polyorganosiloxanes comportant :
- des groupements aminés substitués ou non comme les produits commercialisés sous la dénomination GP 4 Silicone Fluid et GP 7100 par la société GENESEE ou les produits commercialisés sous les dénominations Q2 8220 et DOW CORNING 929 ou 939 par la société DOW CORNING. Les groupements aminés substitués sont en particulier des groupements aminoalkyle en C₁-C₄ ;
- des groupements alcoxylés, comme le produit commercialisé sous la dénomination "SILICONE COPOLYMER F-755" par SWS SILICONES et ABIL WAX® 2428, 2434 et 2440 par la société GOLDSCHMIDT.

De préférence, le ou les corps gras ne comprennent pas de motifs oxyalkylénés, ni de motifs glycérolés.

Le ou les corps gras ne contenant pas de fonction acide carboxylique sont de préférence choisis parmi les alcanes inférieurs en C6-C16, les huiles non siliconées d'origine minérale, végétale ou synthétique, les alcools gras, les esters d'acide gras, les esters d'alcool gras, les silicones.

De préférence, ledit ou lesdits corps gras de la composition selon l'invention sont non siliconés.

Selon un mode de réalisation préféré, ledit ou lesdits corps gras est ou sont choisis parmi l'huile de vaseline, les polydécènes, les esters d'acides gras et/ou d'alcools gras liquides, et leurs mélanges.

Selon un mode de réalisation particulièrement préféré, le corps gras est l'huile de vaseline.

Les compositions (A) et/ou (B) selon la présente invention peuvent comprendre en outre un ou plusieurs tensioactifs.

Dans ce cas, le ou les tensioactifs sont de préférence choisis parmi les tensioactifs non ioniques, ou parmi les tensioactifs anioniques.

Les tensioactifs anioniques sont plus spécialement choisis parmi les sels (en particulier sels de métaux alcalins, notamment de sodium, sels d'ammonium, sels d'amines tels que les sels d'aminoalcools ou sels de métaux alcalino-terreux comme le magnésium) des composés suivants :
- les alkylsulfates, les alkyléthersulfates, alkylamidoéthersulfates, alkylaryl-polyéthersulfates, monoglycérides sulfates ;
- les alkylsulfonates, alkylamidesulfonates, alkylarylsulfonates, α-oléfine-sulfonates, paraffine-sulfonates ;
- les alkylphosphates, les alkylétherphosphates;
- les alkylsulfosuccinates, les alkyléthersulfosuccinates, les alkylamide-sulfosuccinates; les alkylsulfosuccinamates ;
- les alkylsulfoacétates ;
- les acylsarcosinates ; les acyliséthionates et les N-acyltaurates ;
- les sels d'acides gras tels que les acides oléique, ricinoléique, palmitique, stéarique, les acides d'huile de coprah ou d'huile de coprah hydrogénée ;
- les sels d'acides d'alkyl D galactoside uroniques ;
- les acyl-lactylates ;
- les sels des acides alkyléther carboxyliques polyoxyalkylénés, des acides alkylaryléther carboxyliques polyoxyalkylénés, des acides alkylamidoéther carboxyliques polyoxyalkylénés, en particulier ceux comportant de 2 à 50 groupements oxyde d'éthylène ;
- et leurs mélanges.

Il est à noter que le radical alkyle ou acyle de ces différents composés comporte avantageusement de 6 à 24 atomes de carbone, et de préférence de 8 à 24 atomes de carbone, et le radical aryle désignant de préférence un groupement phényle ou benzyle.

Les tensioactifs non ioniques sont plus particulièrement choisis parmi les tensioactifs non ioniques mono ou poly- oxyalkylénés, mono- ou poly- glycérolés. Les motifs oxyalkylénés sont plus particulièrement des motifs oxyéthylénés, oxypropylénés, ou leur combinaison, de préférence oxyéthylénés.

A titre d'exemples de tensioactifs non ioniques oxyalkylénés, on peut citer :
- les alkyl(C₈-C₂₄)phénols oxyalkylénés,
- les alcools en C₈-C₃₀, saturés ou non, linéaires ou ramifiés, oxyalkylénés,
- les amides, en C₈-C₃₀, saturés ou non, linéaires ou ramifiés, oxyalkylénés,
- les esters d'acides en C₈-C₃₀, saturés ou non, linéaires ou ramifiés, et de polyéthylèneglycols,
- les esters d'acides en C₈-C₃₀, saturés ou non, linéaires ou ramifiés, et de sorbitol polyoxyéthylénés,
- les huiles végétales oxyéthylénées, saturées ou non,
- les condensats d'oxyde d'éthylène et/ou d'oxyde de propylène, entre autres,
- et leurs mélanges.

Ces tensioactifs présentent un nombre de moles d'oxyde d'éthylène et/ou de propylène compris entre 1 et 100, de préférence entre 2 et 50. De manière avantageuse, les tensioactifs non ioniques ne comprennent pas de motifs oxypropylénés.

Conformément à un mode de réalisation préféré de l'invention, les tensioactifs non ioniques oxyalkylénés sont choisis parmi les alcools en C₈-C₃₀, oxyéthylénés, les esters d'acides en C₈-C₃₀, saturés ou non, linéaires ou ramifiés et de sorbitol polyoxyéthylénés.

A titre d'exemple de tensioactifs non ioniques mono- ou poly-glycérolés, on utilise de préférence les alcools en C₈-C₄₀, mono- ou poly- glycérolés.

En particulier, les alcools en C₈-C₄₀ mono- ou poly- glycérolés correspondent à la formule suivante :

RO-[CH₂-CH(CH₂OH)-O]ₘ-H

dans laquelle R représente un radical alkyle ou alcényle, linéaire ou ramifié, en C₈-C₄₀, de préférence en C₈-C₃₀, et m représente un nombre allant de 1 à 30 et de préférence de 1 à 10.

A titre d'exemple de composés convenables dans le cadre de l'invention, on peut citer, l'alcool laurique à 4 moles de glycérol (nom INCI : POLYGLYCERYL-4 LAURYL ETHER), l'alcool laurique à 1,5 moles de glycérol, l'alcool oléique à 4 moles de glycérol (nom INCI : POLYGLYCERYL-4 OLEYL ETHER), l'alcool oléique à 2 moles de glycérol (Nom INCI : POLYGLYCERYL-2 OLEYL ETHER), l'alcool cétéarylique à 2 moles de glycérol, l'alcool cétéarylique à 6 moles de glycérol, l'alcool oléocétylique à 6 moles de glycérol, et l'octadécanol à 6 moles de glycérol.

L'alcool peut représenter un mélange d'alcools au même titre que la valeur de m représente une valeur statistique, ce qui signifie que dans un produit commercial peuvent coexister plusieurs espèces d'alcools gras polyglycérolés sous forme d'un mélange.

Parmi les alcools mono- ou poly-glycérolés, on préfère plus particulièrement utiliser l'alcool en C₈/C₁₀ à une mole de glycérol, l'alcool en C₁₀/C₁₂ à 1 mole de glycérol et l'alcool en C₁₂ à 1,5 mole de glycérol.

Selon la présente invention, on préfère employer un ou plusieurs tensioactifs non ioniques.

Selon un mode de réalisation préféré, la composition (A) comprend un ou plusieurs tensioactifs.

Le ou les tensioactifs peuvent être présents dans des proportions allant de 0,1 à 50% en poids, de préférence de 0,5 à 30 % en poids, par rapport au poids total de chaque composition dans laquelle ils sont contenus.

Les compositions (A) et/ou (B) selon la présente invention peuvent également comprendre un ou plusieurs agents épaississants minéraux choisis parmi les argiles organophiles, les silices pyrogénées, ou leurs mélanges.

L'argile organophile peut être choisie parmi la montmorrilonite, la bentonite, l'hectorite, l'attapulgite, la sépiolite, et leurs mélanges. L'argile est de préférence une bentonite ou une hectorite.

Ces argiles peuvent être modifiées avec un composé chimique choisi parmi les amines quaternaires, les amines tertiaires, les acétates aminés, les imidazolines, les savons aminés, les sulfates gras, les alkyl aryl sulfonates, les oxides amines, et leurs mélanges.

Comme argiles organophiles, on peut citer les quaternium-18 bentonites telles que celles vendues sous les dénominations Bentone 3, Bentone 38, Bentone 38V par la société Rhéox, Tixogel VP par la société United catalyst, Claytone 34, Claytone 40, Claytone XL par la société Southern Clay; les stéaralkonium bentonites telles que celles vendues sous les dénominations Bentone 27 par la société Rheox, Tixogel LG par la société United Catalyst, Claytone AF, Claytone APA par la société Southern Clay ; les quaternium-18/benzalkonium bentonite telles que celles vendues sous les dénominations Claytone HT, Claytone PS par la société Southern Clay.

Les silices pyrogénées peuvent être obtenues par hydrolyse à haute température d'un composé volatil du silicium dans une flamme oxhydrique, produisant une silice finement divisée. Ce procédé permet notamment d'obtenir des silices hydrophiles qui présentent un nombre important de groupements silanol à leur surface. De telles silices hydrophiles sont par exemple commercialisées sous les dénominations "AEROSIL 130®", "AEROSIL 200®", "AEROSIL 255®", "AEROSIL 300®", "AEROSIL 380®" par la société Degussa, "CAB-0-SIL HS-5@", "CAB-0-SIL EH-5®", "CAB-0-SIL LM-130®", "CAB-0-SIL MS-55®", "CAB-O-SIL M-5®" par la société Cabot.

Il est possible de modifier chimiquement la surface de la silice par réaction chimique en vue de diminuer le nombre de groupes silanol. On peut notamment substituer des groupes silanol par des groupements hydrophobes : on obtient alors une silice hydrophobe.

Les groupements hydrophobes peuvent être :
- des groupements triméthylsiloxyl, qui sont notamment obtenus par traitement de silice pyrogénée en présence de l'hexaméthyldisilazane. Des silices ainsi traitées sont dénommées "Silica silylate" selon le CTFA (6ème édition, 1995). Elles sont par exemple commercialisées sous les références "AEROSIL R812®" par la société Degussa, "CAB-O-SIL TS-530®" par la société Cabot.
- des groupements diméthylsilyloxyl ou polydiméthylsiloxane, qui sont notamment obtenus par traitement de silice pyrogénée en présence de polydiméthylsiloxane ou du diméthyldichlorosilane. Des silices ainsi traitées sont dénommées "Silica diméthyl silylate" selon le CTFA (6ème édition, 1995). Elles sont par exemple commercialisées sous les références "AEROSIL R972®", "AEROSIL R974®" par la société Degussa, "CAB-O-SIL TS-610®", "CAB-O-SIL TS-720®" par la société Cabot.

La silice pyrogénée présente de préférence une taille de particules pouvant être nanométrique à micrométrique, par exemple allant d'environ de 5 à 200 nm.

Les agents épaississants minéraux préférés sont choisis parmi les hectorites, les bentonites organomodifiées, et les silices pyrogénées éventuellement modifiées.

Lorsqu'il est présent, l'agent épaississant minéral représente de 1 à 30 % en poids par rapport au poids de la composition dans laquelle il est présent.

Les compositions (A) et/ou (B) selon la présente invention peuvent également comprendre un ou plusieurs agents épaississants organiques.

Ces agents épaississants peuvent être choisis parmi les amides d'acides gras (diéthanol- ou monoéthanol-amide de coprah, monoéthanolamide d'acide alkyl éther carboxylique oxyéthyléné), les épaississants polymériques tels que les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique et les polymères associatifs (polymères comprenant des zones hydrophiles, et des zones hydrophobes à chaîne grasse (alkyle, alcényle comprenant au moins 10 atomes de carbone) capables, dans un milieu aqueux, de s'associer réversiblement entre eux ou avec d'autres molécules).

Selon un mode de réalisation particulier, l'épaississant organique est choisi parmi les épaississants cellulosiques (hydroxyéthycellulose, hydroxypropylcellulose, carboxyméthylcellulose), la gomme de guar et ses dérivés (hydroxypropylguar), les gommes d'origine microbienne (gomme de xanthane, gomme de scléroglucane), les homopolymères réticulés d'acide acrylique ou d'acide acrylamidopropanesulfonique, et de préférence parmi les épaississants cellulosiques avec en particulier l' hydroxyéthycellulose.

La teneur en agent(s) épaississant(s) organique(s), s'ils sont présents, varie habituellement de 0,01 % à 20 % en poids, de préférence de 0,1 à 5 % en poids, par rapport au poids de chaque composition dans laquelle ils sont présents.

Avantageusement, la composition (A) se présente sous la forme d'un gel ou d'une crème.

Avantageusement, la composition (B) se présente sous la forme d'une solution, d'une émulsion ou d'un gel.

Selon un premier mode de réalisation de l'invention, la composition (A) comprend en outre un ou plusieurs colorants d'oxydation.

Dans ce cas, l'agent selon l'invention est avantageusement utilisé pour la coloration d'oxydation des fibres kératiniques.

Dans ce mode de réalisation, la composition (A) peut comprendre en outre un plusieurs colorants directs.

Selon un second mode de réalisation de l'invention, la composition (A) comprend en outre un ou plusieurs colorants directs.

Dans ce cas, et lorsque la composition (A) ne comprend pas de colorants d'oxydation, l'agent selon l'invention est avantageusement utilisé pour la coloration directe éclaircissante des fibres kératiniques.

Les colorants d'oxydation utilisables dans la présente invention sont en général choisis parmi les bases d'oxydation, éventuellement combinée(s) à un ou plusieurs coupleurs.

Les bases d'oxydation peuvent être notamment choisies parmi les paraphénylènediamines, les bis-phénylalkylènediamines, les para-aminophénols, les ortho-aminophénols, les bases hétérocycliques et leurs sels d'addition.

Parmi les paraphénylènediamines, on peut citer à titre d'exemple, la paraphénylènediamine, la paratoluylènediamine, la 2-chloro paraphénylènediamine, la 2,3-diméthyl paraphénylènediamine, la 2,6-diméthyl paraphénylènediamine, la 2,6-diéthyl paraphénylènediamine, la 2,5-diméthyl paraphénylènediamine, la N,N-diméthyl paraphénylènediamine, la N,N-diéthyl paraphénylènediamine, la N,N-dipropyl paraphénylènediamine, la 4-amino N,N-diéthyl 3-méthyl aniline, la N,N-bis-(β-hydroxyéthyl) paraphénylènediamine, la 4-N,N-bis-(β-hydroxyéthyl)amino 2-méthyl aniline, la 4-N,N-bis-(β-hydroxyéthyl)amino 2-chloro aniline, la 2-β-hydroxyéthyl paraphénylènediamine, la 2-fluoro paraphénylènediamine, la 2-isopropyl paraphénylènediamine, la N-(β-hydroxypropyl) paraphénylènediamine, la 2-hydroxyméthyl paraphénylènediamine, la N,N-diméthyl 3-méthyl paraphénylènediamine, la N,N-(éthyl, β-hydroxyéthyl) paraphénylènediamine, la N-(β,γ-dihydroxypropyl) paraphénylènediamine, la N-(4'-aminophényl) paraphénylènediamine, la N-phényl paraphénylènediamine, la 2-β-hydroxyéthyloxy paraphénylènediamine, la 2-β-acétylaminoéthyloxy paraphénylènediamine, la N-(β-méthoxyéthyl) paraphénylène-diamine, la 4-aminophénylpyrrolidine, la 2-thiényl paraphénylènediamine, le 2-β hydroxyéthylamino 5-amino toluène, la 3-hydroxy 1-(4'-aminophényl)pyrrolidine et leurs sels d'addition avec un acide.

Parmi les paraphénylènediamines citées ci-dessus, la paraphénylènediamine, la paratoluylènediamine, la 2-isopropyl paraphénylènediamine, la 2-β-hydroxyéthyl paraphénylènediamine, la 2-β-hydroxyéthyloxy paraphénylène-diamine, la 2,6-diméthyl paraphénylènediamine, la 2,6-diéthyl paraphénylènediamine, la 2,3-diméthyl paraphénylènediamine, la N,N-bis-(β-hydroxyéthyl) paraphénylènediamine, la 2-chloro paraphénylènediamine, la 2-β-acétylaminoéthyloxy paraphénylènediamine, et leurs sels d'addition avec un acide sont particulièrement préférées.

Parmi les bis-phénylalkylènediamines, on peut citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthyl-aminophényl) tétraméthylènediamine, la N,N'-bis-(éthyl) N,N'-bis-(4'-amino, 3'-méthylphényl) éthylènediamine, le 1,8-bis-(2,5-diamino phénoxy)-3,6-dioxaoctane, et leurs sels d'addition.

Parmi les para-aminophénols, on peut citer à titre d'exemple, le para-aminophénol, le 4-amino 3-méthyl phénol, le 4-amino 3-fluoro phénol, le 4-amino-3-chlorophénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, le 4-amino 2-fluoro phénol, et leurs sels d'addition avec un acide.

Parmi les ortho-aminophénols, on peut citer à titre d'exemple, le 2-amino phénol, le 2-amino 5-méthyl phénol, le 2-amino 6-méthyl phénol, le 5-acétamido 2-amino phénol, et leurs sels d'addition.

Parmi les bases hétérocycliques, on peut citer à titre d'exemple, les dérivés pyridiniques, les dérivés pyrimidiniques et les dérivés pyrazoliques.

Parmi les dérivés pyridiniques, on peut citer les composés décrits par exemple dans les brevets GB 1 026 978 et GB 1 153 196, comme la 2,5-diamino pyridine, la 2-(4-méthoxyphényl)amino 3-amino pyridine, la 3,4-diamino pyridine, et leurs sels d'addition.

D'autres bases d'oxydation pyridiniques utiles dans la présente invention sont les bases d'oxydation 3-amino pyrazolo-[1,5-a]-pyridines ou leurs sels d'addition décrits par exemple dans la demande de brevet FR 2801308. A titre d'exemple, on peut citer la pyrazolo[1,5-a]pyridin-3-ylamine ; la 2-acétylamino pyrazolo-[1,5-a] pyridin-3-ylamine ; la 2-morpholin-4-yl-pyrazolo[1,5-a]pyridin-3-ylamine ; l'acide 3-amino-pyrazolo[1,5-a]pyridin-2-carboxylique ; la 2-méthoxy-pyrazolo[1,5-a]pyridine-3-ylamino ; le (3-amino-pyrazolo[1,5-a]pyridine-7-yl)-méthanol ; le 2-(3-amino-pyrazolo[1,5-a]pyridine-5-yl)-éthanol ; le 2-(3-amino-pyrazolo[1,5-a]pyridine-7-yl)-éthanol ; le (3-amino-pyrazolo[1,5-a]pyridine-2-yl)-méthanol ; la 3,6-diamino-pyrazolo[1,5-a]pyridine ; la 3,4-diamino-pyrazolo[1,5-a]pyridine ; la pyrazolo[1,5-a]pyridine-3,7-diamine ; la 7-morpholin-4-yl-pyrazolo[1,5-a]pyridin-3-ylamine ; la pyrazolo[1,5-a]pyridine-3,5-diamine ; la 5-morpholin-4-yl-pyrazolo[1,5-a]pyridin-3-ylamine ; le 2-[(3-amino-pyrazolo[1,5-a]pyridin-5-yl)-(2-hydroxyéthyl)-amino]-éthanol ; le 2-[(3-amino-pyrazolo[1,5-a]pyridin-7-yl)-(2-hydroxyéthyl)-amino]-éthanol ; la 3-amino-pyrazolo[1,5-a]pyridine-5-ol ; 3-amino-pyrazolo[1,5-a]pyridine-4-ol ; la 3-amino-pyrazolo[1,5-a]pyridine-6-ol ; la 3-amino-pyrazolo[1,5-a]pyridine-7-ol; ainsi que leurs sels d'addition.

Parmi les dérivés pyrimidiniques, on peut citer les composés décrits par exemple dans les brevets DE 2359399 ; JP 88-169571 ; JP 05-63124 ; EP 0770375 ou demande de brevet WO 96/15765 comme la 2,4,5,6-tétra-aminopyrimidine, la 4-hydroxy 2,5,6-triaminopyrimidine, la 2-hydroxy 4,5,6-triaminopyrimidine, la 2,4-dihydroxy 5,6-diaminopyrimidine, la 2,5,6-triaminopyrimidine et leurs sels d'addition et leurs formes tautomères, lorsqu'il existe un équilibre tautomérique.

Parmi les dérivés pyrazoliques, on peut citer les composés décrits dans les brevets DE 3843892, DE 4133957 et demandes de brevet WO 94/08969, WO 94/08970, FR-A-2 733 749 et DE 195 43 988 comme le 4,5-diamino 1-méthyl pyrazole, le 4,5-diamino 1-(β-hydroxyéthyl) pyrazole, le 3,4-diamino pyrazole, le 4,5-diamino 1-(4'-chlorobenzyl) pyrazole, le 4,5-diamino 1,3-diméthyl pyrazole, le 4,5-diamino 3-méthyl 1-phényl pyrazole, le 4,5-diamino 1-méthyl 3-phényl pyrazole, le 4-amino 1,3-diméthyl 5-hydrazino pyrazole, le 1-benzyl 4,5-diamino 3-méthyl pyrazole, le 4,5-diamino 3-tert-butyl 1-méthyl pyrazole, le 4,5-diamino 1-tert-butyl 3-méthyl pyrazole, le 4,5-diamino 1-(β-hydroxyéthyl) 3-méthyl pyrazole, le 4,5-diamino 1-éthyl 3-méthyl pyrazole, le 4,5-diamino 1-éthyl 3-(4'-méthoxyphényl) pyrazole, le 4,5-diamino 1-éthyl 3-hydroxyméthyl pyrazole, le 4,5-diamino 3-hydroxyméthyl 1-méthyl pyrazole, le 4,5-diamino 3-hydroxyméthyl 1-isopropyl pyrazole, le 4,5-diamino 3-méthyl 1-isopropyl pyrazole, le 4-amino 5-(2'-aminoéthyl)amino 1,3-diméthyl pyrazole, le 3,4,5-triamino pyrazole, le 1-méthyl 3,4,5-triamino pyrazole, le 3,5-diamino 1-méthyl 4-méthylamino pyrazole, le 3,5-diamino 4-(β-hydroxyéthyl)amino 1-méthyl pyrazole, et leurs sels d'addition. On peut aussi utiliser le 4-5-diamino 1-(β-méthoxyéthyl)pyrazo le.

De préférence, on utilisera un 4,5-diaminopyrazole et encore plus préférentiellement le 4,5-diamino-1-(β-hydroxyéthyl)-pyrazole et/ou l'un des ses sels.

A titre de dérivés pyrazoliques, on peut également citer les diamino N,N-dihydropyrazolopyrazolones et notamment celles décrites dans la demande FR-A-2 886 136 telles que les composés suivants et leurs sels d'addition : 2,3-diamino-6,7-dihydro-1H,5H-pyrazolo[1,2-a]pyrazol-1-one, 2-amino-3-éthylamino-6,7-dihydro-1H,5H-pyrazolo[1,2-a]pyrazol-1-one, 2-amino-3-isopropylamino-6,7-dihydro-1H,5H-pyrazolo[1,2-a]pyrazol-1-one, 2-amino-3-(pyrrolidin-1-yl)-6,7-dihydro-1H,5H-pyrazolo[1,2-a]pyrazol-1-one, 4,5-diamino-1,2-diméthyl-1,2-dihydro-pyrazol-3-one, 4,5-diamino-1,2-diéthyl-1,2-dihydro-pyrazol-3-one, 4,5-diamino-1,2-di-(2-hydroxyéthyl)-1,2-dihydro-pyrazol-3-one, 2-amino-3-(2-hydroxyéthyl)amino-6,7-dihydro-1H,5H-pyrazolo[1,2-a]pyrazol-1-one, 2-amino-3-diméthylamino-6,7-dihydro-1H,5H-pyrazolo[1,2-a]pyrazol-1-one, 2,3-diamino-5,6,7,8-tétrahydro-1H,6H-pyridazino[1,2-a]pyrazol-1-one, 4-amino-1,2-diéthyl-5-(pyrrolidin-1-yl)-1,2-dihydro-pyrazol-3-one, 4-amino-5-(3-diméthylamino-pyrrolidin-1-yl)-1,2-diéthyl-1,2-dihydro-pyrazol-3-one, 2,3-diamino-6-hydroxy-6,7-dihydro-1H,5H-pyrazolo[1,2-a]pyrazol-1-one.

On préférera utiliser la 2,3-diamino-6,7-dihydro-1H,5H-pyrazolo[1,2-a]pyrazol-1-one et/ou un de ses sels.

A titre de bases hétérocycliques, on utilisera préférentiellement le 4,5-diamino-1-(β-hydroxyéthyl)pyrazole et/ou la 2,3-diamino-6,7-dihydro-1H,5H-pyrazolo[1,2-a]pyrazol-1-one et/ou un de leurs sels.

Les coupleurs utilisables dans la présente invention peuvent être choisis parmi ceux conventionnellement utilisés pour la teinture des fibres kératiniques.

Parmi ces coupleurs, on peut notamment citer les méta-phénylènediamines, les méta-aminophénols, les méta-diphénols, les coupleurs naphtaléniques, les coupleurs hétérocycliques ainsi que leurs sels d'addition.

A titre d'exemple, on peut citer le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxybenzène, le 1,3-diamino benzène, le 1,3-bis-(2,4-diaminophénoxy) propane, la 3-uréido aniline, le 3-uréido 1-diméthylamino benzène, le sésamol, le 1-β-hydroxyéthylamino-3,4-méthylènedioxybenzène, l'α-naphtol, le 2-méthyl-1-naphtol, le 6-hydroxy indole, le 4-hydroxy indole, le 4-hydroxy N-méthyl indole, la 2-amino-3-hydroxy pyridine, la 6- hydroxy benzomorpholine la 3,5-diamino-2,6-diméthoxypyridine, le 1-N-(β-hydroxyéthyl)amino-3,4-méthylène dioxybenzène, le 2,6-bis-(β-hydroxyéthylamino)toluène, la 6-hydroxy indoline, la 2,6-dihydroxy 4-méthyl pyridine, la 1-H 3-méthyl pyrazole 5-one, la 1-phényl 3-méthyl pyrazole 5-one, le 2,6-diméthyl pyrazolo [1,5-b]-1,2,4-triazole, le 2,6-diméthyl [3,2-c]-1,2,4-triazole, le 6-méthyl pyrazolo [1,5-a]-benzimidazole, leurs sels d'addition avec un acide, et leurs mélanges.

D'une manière générale, les sels d'addition des bases d'oxydation et des coupleurs utilisables dans le cadre de l'invention sont notamment choisis parmi les sels d'addition avec un acide tels que les chlorhydrates, les bromhydrates, les sulfates, les citrates, les succinates, les tartrates, les lactates, les tosylates, les benzènesulfonates, les phosphates et les acétates.

La ou les bases d'oxydation peuvent représenter chacune avantageusement de 0,001 à 10 % en poids par rapport au poids total de la composition (A), et de préférence de 0,005 à 5 % en poids par rapport au poids total de cette composition.

Le ou les coupleurs, s'ils sont présents, peuvent représenter chacun avantageusement de 0,001 à 10 % en poids par rapport au poids total de la composition (A), et de préférence de 0,005 à 5 % en poids par rapport au poids total de cette composition.

Les colorants directs susceptibles d'être employés dans la composition (A) sont plus particulièrement choisis parmi les espèces ioniques ou non ioniques, de préférence cationiques ou non ioniques.

A titre d'exemples de colorants directs convenables, on peut citer les colorants directs azoïques ; méthiniques ; carbonyles ; aziniques ; nitrés (hétéro)aryle ; tri-(hétéro)aryle méthanes ; les porphyrines ; les phtalocyanines et les colorants directs naturels, seuls ou en mélanges.

Plus particulièrement, les colorants azoïques comprennent une fonction -N=N- dont les deux atomes d'azote ne sont pas simultanément engagés dans un cycle. Il n'est toutefois pas exclu que l'un des deux atomes d'azote de l'enchaînement -N=N- soit engagé dans un cycle.

Les colorants de la famille des méthines sont plus particulièrement des composés comprenant au moins un enchaînement choisi parmi >C=C< et -N=C< dont les deux atomes ne sont pas simultanément engagés dans un cycle. Il est toutefois précisé que l'un des atomes d'azote ou de carbone des enchaînements peut être engagé dans un cycle. Plus particulièrement, les colorants de cette famille sont issus de composés de type méthine, azométhine, mono- et di-arylméthane, indoamines (ou diphénylamines), indophénols, indoanilines, carbocyanines, azacabocyanines et leurs isomères, diazacarbocyanines et leurs isomères, tétraazacarbocyanines, hémicyanines.

Concernant les colorants de la famille des carbonyles, on peut citer par exemple les colorants choisis parmi les acridone, benzoquinone, anthraquinone, naphtoquinone, benzanthrone, anthranthrone, pyranthrone, pyrazolanthrone, pyrimidinoanthrone, flavanthrone, idanthrone, flavone, (iso)violanthrone, isoindolinone, benzimidazolone, isoquinolinone, anthrapyridone, pyrazoloquinazolone, périnone, quinacridone, quinophthalone, indigoïde, thioindigo, naphtalimide, anthrapyrimidine, dicétopyrrolopyrrole, coumarine.

Concernant les colorants de la famille des azines cycliques, on peut citer notamment les azine, xanthène, thioxanthène, fluorindine, acridine, (di)oxazine, (di)thiazine, pyronine.

Les colorants nitrés (hétéro)aromatiques sont plus particulièrement des colorants directs nitrés benzéniques ou nitrés pyridiniques.

Concernant les colorants de type porphyrines ou phtalocyanines, on peut mettre en oeuvre des composés cationiques ou non, comprenant éventuellement un ou plusieurs métaux ou ions métalliques, comme par exemple des métaux alcalins et alcalino-terreux, le zinc et le silicium.

A titre d'exemple de colorants directs particulièrement convenables, on peut citer les colorants nitrés de la série benzénique ; les colorants directs azoïques ; azométhiniques ; méthiniques ; les azacarbocyanines comme les tétraazacarbocyanines (tétraazapentaméthines) ; les colorants directs quinoniques et en particulier anthraquinoniques, naphtoquinoniques ou benzoquinoniques ; les colorants directs aziniques ; xanthéniques ; triarylméthaniques ; indoaminiques ; indigoïdes ; phtalocyanines, porphyrines et les colorants directs naturels, seuls ou en mélanges.

Ces colorants peuvent être des colorants monochromophoriques (c'est-à-dire ne comprenant qu'un seul colorant) ou polychromophoriques, de préférence di- ou tri- chromophoriques ; les chromophores pouvant être identiques ou non, de la même famille chimique ou non. A noter que qu'un colorant polychromophorique comprend plusieurs radicaux issus chacun d'une molécule absorbant dans le domaine visible entre 400 et 800 nm. De plus cette absorbance du colorant ne nécessite ni oxydation préalable de celui-ci, ni association avec d'autre(s) espèce(s) chimique(s).

Dans le cas de colorants polychromophoriques, les chromophores sont reliés entre eux au moyen d'au moins un bras de liaison qui peut être cationique ou non.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

Si les hétérocycles ou noyaux aromatiques sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ; un radical amino ; un radical amino substitué par un ou deux radicaux alkyle, identiques ou différents, en C₁-C₄ éventuellement porteurs d'au moins un groupement hydroxyle.

Parmi les colorants directs benzéniques utilisables selon l'invention, on peut citer de manière non limitative les composés suivants :
- 1,4-diamino-2-nitrobenzène,
- 1-amino-2 nitro-4-β- hydroxyéthylaminobenzène
- 1-amino-2 nitro-4-bis(β-hydroxyéthyl)-aminobenzène
- 1,4-bis(β-hydroxyéthylamino)-2-nitrobenzène
- 1-β-hydroxyéthylamino-2-nitro-4-bis-(β-hydroxyéthylamino)-benzène
- 1-β-hydroxyéthylamino-2-nitro-4-aminobenzène
- 1-β-hydroxyéthylamino-2-nitro-4-(éthyl)(β-hydroxyéthyl)-aminobenzène
- 1-amino-3-méthyl-4-β-hydroxyéthylamino-6-nitrobenzène
- 1-amino-2-nitro-4-β-hydroxyéthylamino-5-chlorobenzène
- 1,2-diamino-4-nitrobenzène
- 1-amino-2-β-hydroxyéthylamino-5-nitrobenzène
- 1,2-bis-(β-hydroxyéthylamino)-4-nitrobenzène
- 1-amino-2-tris-(hydroxyméthyl)-méthylamino-5-nitrobenzène
- 1-Hydroxy-2-amino-5-nitrobenzène
- 1-Hydroxy-2-amino-4-nitrobenzène
- 1-Hydroxy-3-nitro-4-aminobenzène
- 1-Hydroxy-2-amino-4,6-dinitrobenzène
- 1-β-hydroxyéthyloxy-2-β-hydroxyéthylamino-5-nitrobenzène
- 1-Méthoxy-2-β-hydroxyéthylamino-5-nitrobenzène
- 1-β-hydroxyéthyloxy-3-méthylamino-4-nitrobenzène
- 1-β,γ-dihydroxypropyloxy-3-méthylamino-4-nitrobenzène
- 1-β-hydroxyéthylamino-4-β,γ-dihydroxypropyloxy-2-nitrobenzène
- 1-β,γ-dihydroxypropylamino-4-trifluorométhyl-2-nitrobenzène
- 1-β-hydroxyéthylamino-4-trifluorométhyl-2-nitrobenzène
- 1-β-hydroxyéthylamino-3-méthyl-2-nitrobenzène
- 1-β-aminoéthylamino-5-méthoxy-2-nitrobenzène
- 1-Hydroxy-2-chloro-6-éthylamino-4-nitrobenzène
- 1-Hydroxy-2-chloro-6-amino-4-nitrobenzène
- 1-Hydroxy-6-bis-(β-hydroxyéthyl)-amino-3-nitrobenzène
- 1-β-hydroxyéthylamino-2-nitrobenzène
- 1-Hydroxy-4-β-hydroxyéthylamino-3-nitrobenzène.

Parmi les colorants directs azoïques, azométhines, méthines ou tétraazapentaméthines utilisables selon l'invention on peut citer les colorants cationiques décrits dans les demandes de brevets WO 95/15144, WO 95/01772 et EP 714954 ; FR 2189006, FR 2285851, FR-2140205, EP 1378544, EP 1674073.

Ainsi, on peut tout notamment citer les colorants suivants de formules (I) à (IV) ci-après, et de préférence les composés de formules (I) et (III) suivantes : 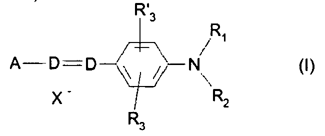 dans laquelle :
D représente un atome d'azote ou le groupement -CH,
R₁ et R₂, identiques ou différents, représentent un atome d'hydrogène ; un radical alkyle en C₁-C₄ pouvant être substitué par un radical -CN, -OH ou -NH₂ ou forment avec un atome de carbone du cycle benzénique un hétérocycle éventuellement oxygéné ou azoté, pouvant être substitué par un ou plusieurs radicaux alkyle en C₁-C₄ ; un radical 4'-aminophényle,
R₃ et R'₃, identiques ou différents, représentent un atome d'hydrogène ou d'halogène choisi parmi le chlore, le brome, l'iode et le fluor, un radical cyano, alkyl en C₁-C₄, alcoxy en C₁-C₄ ou acétyloxy,
X - représente un anion de préférence choisi parmi le chlorure, le méthyl sulfate et l'acétate,
A représente un groupement choisi par les structures A1 à A18, de préférence A1, A4, A7, A13 et A18 suivantes : 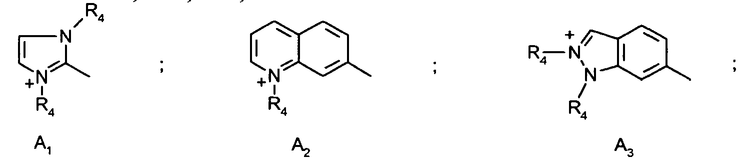 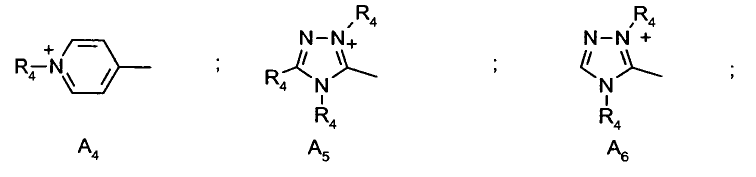 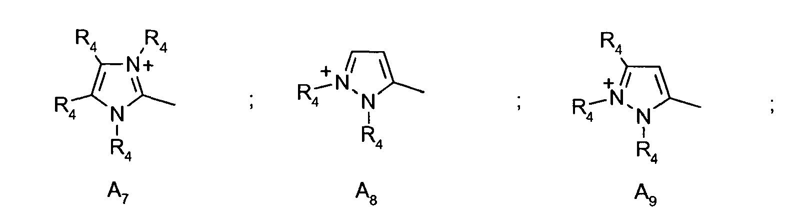 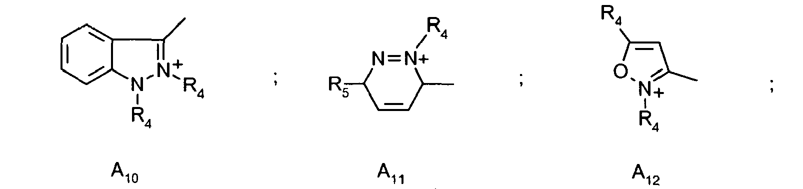 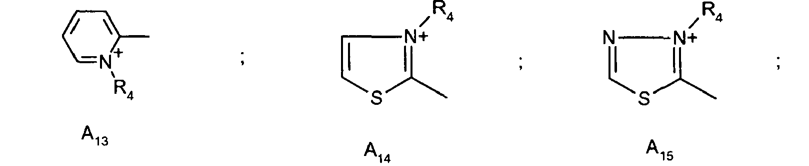 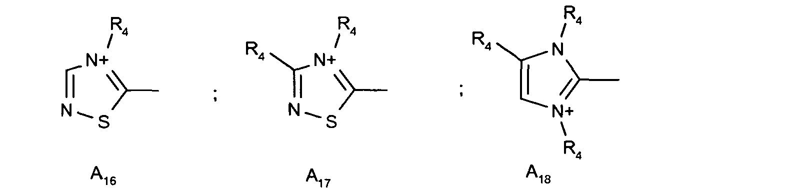
dans lesquelles R₄ représente un radical alkyle en C₁-C₄ pouvant être substitué par un radical hydroxyle et R₅ représente un radical alcoxy en C₁-C₄ ; 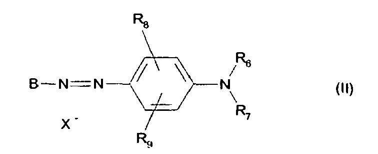
dans laquelle :
R₆ représente un atome d'hydrogène ou un radical alkyle en C₁-C₄,
R₇ représente un atome d'hydrogène, un radical alkyle pouvant être substitué par un radical -CN ou par un groupement amino, un radical 4'-aminophényle ou forme avec R₆ un hétérocycle éventuellement oxygéné et/ou azoté pouvant être substitué par un radical alkyle en C₁-C₄,
R₈ et R₉, identiques ou différents, représentent un atome d'hydrogène, un atome d'halogène tel que le brome, le chlore, l'iode ou le fluor, un radical alkyle en C₁-C₄ ou alcoxy en C₁-C₄, un radical - CN,
X - représente un anion de préférence choisi parmi le chlorure, le méthyl sulfate et l'acétate,
B représente un groupement choisi par les structures B1 à B6 suivantes : 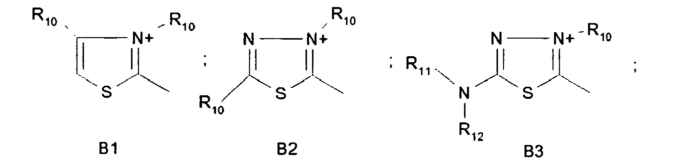 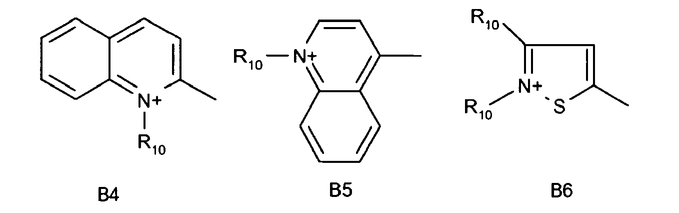
dans lesquelles R₁₀ représente un radical alkyle en C₁-C₄, R₁₁ et R₁₂, identiques ou différents, représentent un atome d'hydrogène ou un radical alkyle en C₁-C₄ ; 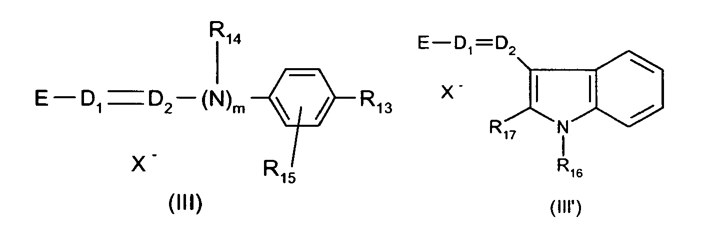
dans lesquelles :
R₁₃ représente un atome d'hydrogène, un radical alcoxy en C₁-C₄, un atome d'halogène tel que le brome, le chlore, l'iode ou le fluor,
R₁₄ représente un atome d'hydrogène, un radical alkyle en C₁-C₄ ou forme avec un atome de carbone du cycle benzénique un hétérocycle éventuellement oxygéné et/ou substitué par un ou plusieurs groupements alkyle en C₁-C₄,
R₁₅ représente un atome d'hydrogène ou d'halogène tel que le brome, le chlore, l'iode ou le fluor,
R₁₆ et R₁₇, identiques ou différents, représentent un atome d'hydrogène ou un radical alkyle en C₁-C₄,
D₁ et D₂, identiques ou différents, représentent un atome d'azote ou le groupement -CH,
m = 0 ou 1, de préférence 1,
étant entendu que lorsque R₁₃ représente un groupement amino non substitué, alors D₁ et D₂ représentent simultanément un groupement -CH et m = 0,
X - représente un anion de préférence choisi parmi le chlorure, le méthyl sulfate et l'acétate,
E représente un groupement choisi par les structures E1 à E8, plus particulièrement E1, E2 et E7, suivantes : 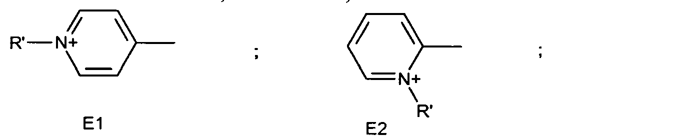 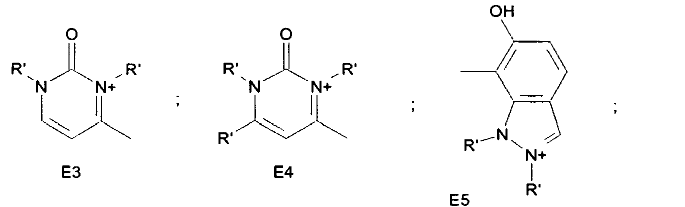 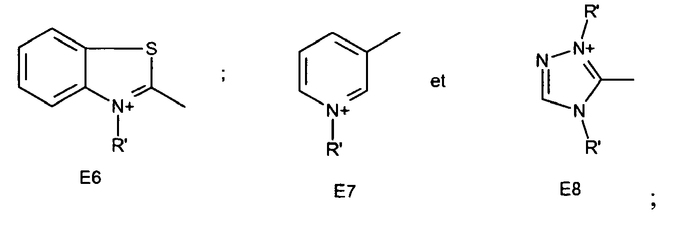
dans lesquelles R' représente un radical alkyle en C₁-C₄ ;
lorsque m = 0 et que D₁ représente un atome d'azote, alors E peut également désigner un groupement de structure E9 suivante : 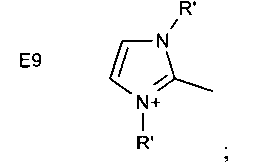
dans laquelle R' représente un radical alkyle en C₁-C₄.

   G——N==N——J (IV)
dans laquelle :
le symbole G représente un groupement choisi parmi les structures G₁ à G₃ suivantes : 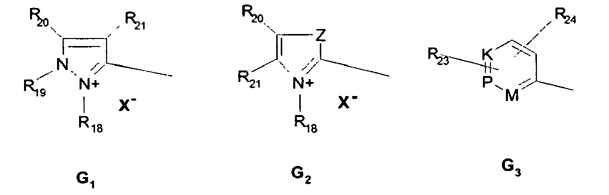 structures G₁ à G₃ dans lesquelles,
R₁₈ désigne un radical alkyle en C₁-C₄, un radical phényle pouvant être substitué par un radical alkyle en C₁-C₄ ou un atome d'halogène choisi parmi le chlore, le brome, l'iode et le fluor ;
R₁₉ désigne un radical alkyle en C₁-C₄ ou un radical phényle;
R₂₀ et R₂₁, identiques ou différents, représentent un radical alkyle en C₁-C₄, un radical phényle, ou forment ensemble dans G₁ un cycle benzénique substitué par un ou plusieurs radicaux alkyle en C₁-C₄, alcoxy en C₁-C₄, ou NO₂, ou forment ensemble dans G₂ un cycle benzénique éventuellement substitué par un ou plusieurs radicaux alkyle en C₁-C₄, alcoxy en C₁-C₄, ou NO₂ ;
R₂₀ peut désigner en outre un atome d'hydrogène ;
Z désigne un atome d'oxygène, de soufre ou un groupement - NR₁₉ ;
M représente un groupement -CH, -CR (R désignant alkyle en C₁-C₄),
ou -NR₂₂(X⁻)ᵣ;
K représente un groupement -CH, -CR (R désignant alkyle en C₁-C₄),
ou -NR₂₂(X⁻)ᵣ ;
P représente un groupement -CH, -CR (R désignant alkyle en C₁-C₄),
ou -NR₂₂(X⁻)ᵣ; r désigne zéro ou 1;
R₂₂ représente un atome O⁻, un radical alcoxy en C₁-C₄, ou un radical alkyle en C₁-C₄;
R₂₃ et R₂₄, identiques ou différents, représentent un atome d'hydrogène ou d'halogène choisi parmi le chlore, le brome, l'iode et le fluor, un radical alkyle en C₁-C₄, alcoxy en C₁-C₄ , un radical -NO₂ ;
X⁻ représente un anion de préférence choisi parmi le chlorure, l'iodure, le méthyl sulfate, l'éthyl sulfate, l'acétate et le perchlorate;
sous réserve que,
si R₂₂ désigne O⁻, alors r désigne zéro;
si K ou P ou M désignent -N-alkyle C₁-C₄ X⁻, alors R₂₃ ou R₂₄ est de préférence différent d'un atome d'hydrogène;
si K désigne -NR₂₂(X⁻)ᵣ , alors M= P= -CH, -CR;
si M désigne -NR₂₂(X⁻)ᵣ , alors K= P= -CH, -CR;
si P désigne -NR₂₂(X⁻)ᵣ , alors K= M et désignent -CH ou -CR;
si Z désigne un atome de soufre avec R₂₁ désignant alkyle en C₁-C₄, alors R₂₀ est différent d'un atome d'hydrogène;
si Z désigne -NR₂₂ avec R₁₉ désignant alkyle en C₁-C₄, alors au moins l'un des radicaux R₁₈, R₂₀ ou R₂₁ du groupement de structure G₂ est différent d'un radical alkyle en C₁-C₄;
le symbole J représente :
   - (a) un groupement de structure J₁ suivante : 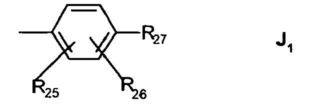 structure J₁ dans laquelle,
      R₂₅ représente un atome d'hydrogène, un atome d'halogène choisi parmi le chlore, le brome, l'iode et le fluor, un radical alkyle en C₁-C₄, alcoxy en C₁-C₄, un radical -OH, -NO₂, -NHR₂₈, -NR₂₉R₃₀, -NHCO alkyle en C₁-C₄, ou forme avec R₂₆ un cycle à 5 ou 6 chaînons contenant ou non un ou plusieurs hétéroatomes choisis parmi l'azote, l'oxygène ou le soufre;
      R₂₆ représente un atome d'hydrogène, un atome d'halogène choisi parmi le chlore, le brome, l'iode et le fluor, un radical alkyle en C₁-C₄, alcoxy en C₁-C₄,
      ou forme avec R₂₇ ou R₂₈ un cycle à 5 ou 6 chaînons contenant ou non un ou plusieurs hétéroatomes choisis parmi l'azote, l'oxygène ou le soufre;
      R₂₇ représente un atome d'hydrogène, un radical -OH, un radical -NHR₂₈, un radical -NR₂₉R₃₀;
      R₂₈ représente un atome d'hydrogène, un radical alkyle en C₁-C₄, un radical monohydroxyalkyle en C₁-C₄, polyhydroxyalkyle en C₂-C₄, un radical phényle;
      R₂₉ et R₃₀, identiques ou différents, représentent un radical alkyle en C₁-C₄, un radical monohydroxyalkyle en C₁-C₄, polyhydroxyalkyle en C₂-C₄;
   - (b) un groupement hétérocyclique azoté à 5 ou 6 chaînons susceptible de renfermer d'autres hétéroatomes et/ou des groupements carbonylés et pouvant être substitué par un ou plusieurs radicaux alkyle en C₁-C₄, amino ou phényle,
et notamment un groupement de structure J₂ suivante : 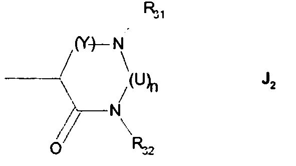 structure J₂ dans laquelle,
R₃₁ et R₃₂, identiques ou différents, représentent un atome d'hydrogène, un radical alkyle en C₁-C₄, un radical phényle;
Y désigne le radical -CO- ou le radical 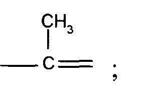
n = 0 ou 1, avec, lorsque n désigne 1, U désigne le radical - CO-.

Dans les structures des colorants (I) à (IV) définies ci-dessus, le groupement alkyle ou alcoxy en C₁-C₄ désigne de préférence méthyle, éthyle, butyle, méthoxy, éthoxy.

Parmi les colorants de formules (I) et (III), on préfère les composés suivants :

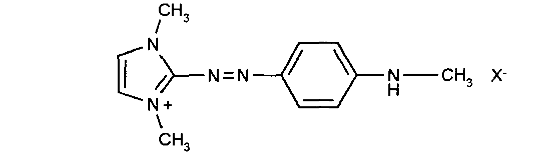

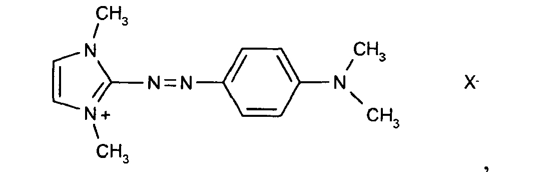

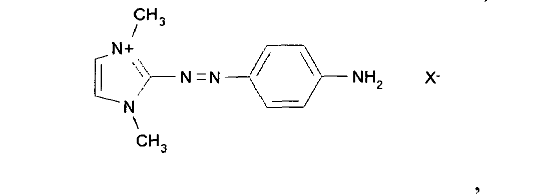

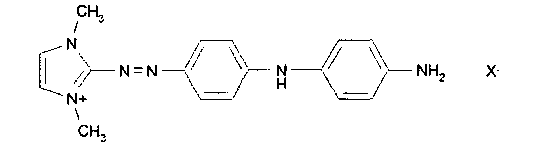

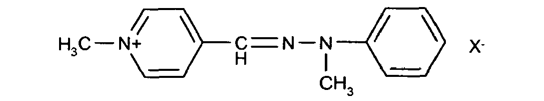

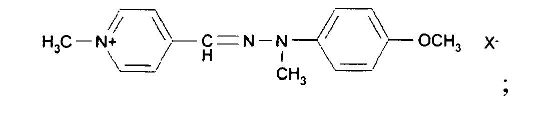

On peut également citer parmi les colorants directs azoïques les colorants suivants, décrits dans le COLOUR INDEX INTERNATIONAL 3e édition :
- Disperse Red 17
- Basic Red 22
- Basic Red 76
- Basic Yellow 57
- Basic Brown 16
- Basic Brown 17
- Disperse Black 9.

On peut aussi citer le 1-(4'-aminodiphénylazo)-2-méthyl-4bis-(β-hydroxyéthyl) aminobenzène.

Parmi les colorants directs quinoniques on peut citer les colorants suivants :
- Disperse Red 15
- Solvent Violet 13
- Disperse Violet 1
- Disperse Violet 4
- Disperse Blue 1
- Disperse Violet 8
- Disperse Blue 3
- Disperse Red 11 1
- Disperse Blue 7
- Basic Blue 22
- Disperse Violet 15
- Basic Blue 99
ainsi que les composés suivants :
-1-N-méthylmorpholiniumpropylamino-4-hydroxy anthraquinone
- 1-Aminopropylamino-4-méthylaminoanthraquinone
- 1-Aminopropylaminoanthraquinone
- 5-β-hydroxyéthyl-1,4-diaminoanthraquinone
- 2-Aminoéthylaminoanthraquinone
- 1,4-Bis-(β,γ-dihydroxypropylamino)-anthraquinone.

Parmi les colorants aziniques, on peut citer les composés suivants :
- Basic Blue 17
- Basic Red 2.

Parmi les colorants triarylméthaniques utilisables selon l'invention, on peut citer les composés suivants :
- Basic Green 1
- Basic Violet 3
- Basic Violet 14
- Basic Blue 7
- Basic Blue 26

Parmi les colorants indoaminiques utilisables selon l'invention, on peut citer les composés suivants :
- 2-β-hydroxyéthlyamino-5-[bis-(β-4'-hydroxyéthyl)amino]anilino-1,4-benzoquinone
- 2-β-hydroxyéthylamino-5-(2'-méthoxy-4'-amino)anilino-1,4-benzoquinone
- 3-N(2'-Chloro-4'-hydroxy)phényl-acétylamino-6-méthoxy-1,4-benzoquinone imine
- 3-N(3'-Chloro-4'-méthylamino)phényl-uréido-6-méthyl-1,4-benzoquinone imine
- 3-[4'-N-(Ethyl,carbamylméthyl)-amino]-phényl-uréido-6-méthyl-1,4-benzoquinone imine.

Parmi les colorants de type tétraazapentaméthiniques utilisables selon l'invention, on peut citer les composés suivants figurant dans le tableau ci-dessous:

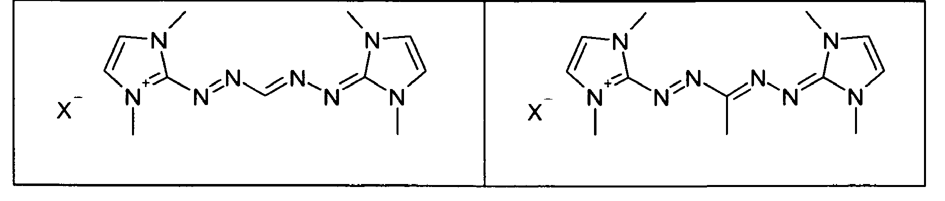

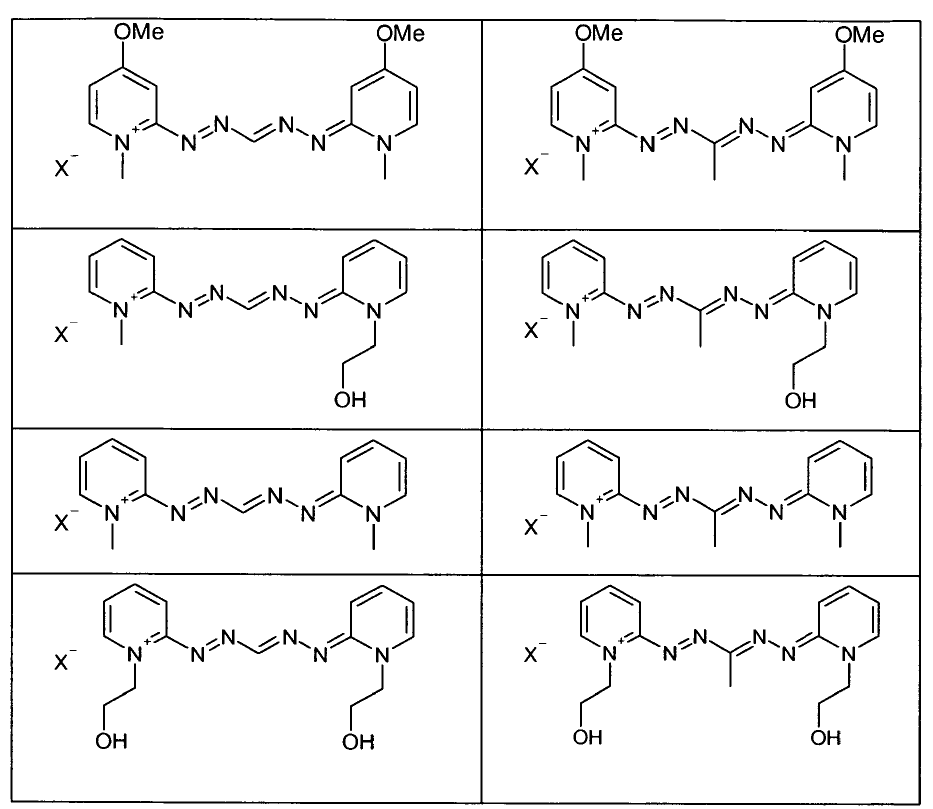

X représente un anion de préférence choisi parmi le chlorure, l'iodure, le méthyl sulfate, l'éthyl sulfate, l'acétate et le perchlorate.

Parmi les colorants polychromophoriques, on peut citer plus particulièrement les colorants di- ou tri- chromophoriques azoïques et/ou azométhiniques (hydrazoniques), symétriques ou non, comprenant d'une part au moins un hétérocycle aromatique comprenant 5 ou 6 chaînons, éventuellement condensé, comprenant au moins un atome d'azote quaternisé engagé dans ledit hétérocycle et éventuellement au moins un autre hétéroatome (tel que l'azote, le soufre, l'oxygène), et d'autre part, au moins un groupement phényle ou naphtyle, éventuellement substitué, éventuellement porteur d'au moins un groupement OR avec R représentant un atome d'hydrogène, un radical alkyle éventuellement substitué en C₁-C₆, un noyau phényle éventuellement substitué, ou d'au moins un groupement N(R')₂ avec R' identiques ou non, représentant un atome d'hydrogène, un radical alkyle éventuellement substitué en C₁-C₆, un noyau phényle éventuellement substitué ; les radicaux R' pouvant former avec l'atome d'azote auquel ils sont liés, un hétérocycle saturé à 5 ou 6 chaînons, ou bien encore l'un et/ou les deux radicaux R' peuvent former chacun avec l'atome de carbone du cycle aromatique placé en ortho de l'atome d'azote, un hétérocycle saturé à 5 ou 6 chaînons.

A titre d'hétérocycle cationique aromatique, on peut citer de préférence, les cycles à 5 ou 6 chaînons comprenant 1 à 3 atomes d'azote, de préférence 1 ou 2 atomes d'azote, l'un étant quaternisé ; ledit hétérocycle étant par ailleurs éventuellement condensé à un noyau benzénique. Il est à noter de même que l'hétérocycle peut éventuellement comprendre un autre hétéroatome différent de l'azote, comme le soufre ou l'oxygène.

Si les hétérocycles ou groupements phényle ou napthyle sont substitués, ils le sont par exemple par un ou plusieurs radicaux alkyle en C₁-C₈ éventuellement substitués par un groupement hydroxy, alcoxy en C₁-C₂, hydroxyalcoxy en C₂-C₄, acétylamino, amino substitué par un ou deux radicaux alkyle en C₁-C₄, éventuellement porteurs d'au moins un groupement hydroxyle ou les deux radicaux pouvant former avec l'atome d'azote auquel ils sont rattachés, un hétérocycle à 5 ou 6 chaînons, comprenant éventuellement un autre hétéroatome identique ou différent de l'azote ; un atome d'halogène ; un groupement hydroxyle ; un radical alcoxy en C₁-C₂ ; un radical hydroxyalcoxy en C₂-C₄ ;un radical amino ; un radical amino substitué par un ou deux radicaux alkyle, identiques ou différents, en C₁-C₄ éventuellement porteurs d'au moins un groupement hydroxyle.

Ces polychromophores sont reliés entre eux au moyen d'au moins un bras de liaison comprenant éventuellement au moins un atome d'azote quaternisé engagé ou non dans un hétérocycle saturé ou non, éventuellement aromatique.

De préférence, le bras de liaison est une chaîne alkyle en C₁-C₂₀, linéaire, ramifiée ou cyclique, éventuellement interrompue par au moins un hétéroatome (tel que l'azote, l'oxygène) et/ou par au moins un groupe en comprenant (CO, SO₂), éventuellement interrompue par au moins un hétérocycle condensé ou non avec un noyau phényle et comprenant au moins un atome d'azote quaternisé engagé dans ledit cycle et éventuellement au moins un autre hétéroatome (tel que l'oxygène, l'azote ou le soufre), éventuellement interrompue par au moins un groupement phényle ou naphtyle substitué ou non, éventuellement au moins un groupement ammonium quaternaire substitué par deux groupements alkyle en C₁-C₁₅ éventuellement substitués ; le bras de liaison ne comprenant pas de groupement nitro, nitroso ou peroxo.

La liaison entre le bras de liaison et chaque chromophore se fait en général au moyen d'un hétéroatome substituant le noyau phényle ou napthyle ou au moyen de l'atome d'azote quaternisé de l'hétérocycle cationique.

Le colorant peut comprendre des chromophores identiques ou non.

A titre d'exemples de tels colorants, on pourra notamment se reporter aux demandes de brevets EP 1637566, EP 1619221, EP 1634926, EP 1619220, EP 1672033, EP 1671954, EP 1671955, EP 1679312, EP 1671951, EP167952, EP167971, WO 06/063866, WO 06/063867, WO 06/063868, WO 06/063869, EP 1408919, EP 1377264, EP 1377262, EP 1377261, EP 1377263, EP 1399425, EP 1399117, EP 1416909, EP 1399116, EP 1671560.

On peut aussi également mettre en oeuvre des colorants directs cationiques cités dans les demandes EP 1006153, qui décrit des colorants comprenant deux chromophores de type anthraquinones reliés au moyen d'un bras de liaison cationique ; EP 1433472, EP 1433474, EP 1433471 et EP 1433473 qui décrivent des colorants dichromophoriques identiques ou non, reliés par un bras de liaison cationique ou non, ainsi que EP 6291333 qui décrit notamment des colorants comprenant trois chromophores, l'un d'eux étant un chromophore anthraquinone auquel sont reliés deux chromophores de type azoïque ou diazacarbocyanine ou l'un de ses isomères.

Parmi les colorants directs naturels utilisables selon l'invention, on peut citer la lawsone, la juglone, l'alizarine, la purpurine, l'acide carminique, l'acide kermésique, la purpurogalline, le protocatéchaldéhyde, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Lorsqu'ils sont présents, le ou les colorants directs représentent avantageusement de 0,001 à 10% en poids du poids total de la composition (A), et de préférence de 0,005 à 5% en poids.

Selon un troisième mode de réalisation de l'invention, les compositions (A) et (B) ne comprennent pas de colorant direct ni de colorant d'oxydation (bases et coupleurs) ou bien, s'ils sont présents, leur teneur totale ne dépasse pas 0,005% en poids par rapport au poids total de chaque composition.

Dans ce mode de réalisation, l'agent selon l'invention est avantageusement utilisé pour la décoloration des fibres kératiniques.

Dans ce mode de réalisation, la composition (A) peut avantageusement comprendre un ou plusieurs adjuvants solides ou pâteux, et de préférence pulvérulents. Les adjuvants peuvent alors être choisis parmi les argiles, les sels différents des sels d'ammonium, les tensioactifs anioniques, non ioniques, cationiques ou zwittérioniques, les épaississants naturels ou de synthèse, l'amidon éventuellement modifié, les billes de verre, la silice, le nylon, l'alumine, le dioxyde de titane, les zéolithes, le poly(méthacrylate de méthyle) (PMMA), le chitosane, la maltodextrine, la cyclodextrine, les mono- ou disaccharides comme le glucose, le saccharose, le sorbitol ou le fructose, l'oxyde de zinc, de zirconium, les silicabades, le talc, les borosilicates notamment de calcium, le polyéthylène, le polytétrafluoroéthylène (PTFE), la cellulose et ses dérivés, les composés superabsorbants, les carbonates de magnésium ou de calcium, le polyacrylamide, l'hydroxyapatite poreux, la sciure de bois, la poudre de fucus, la polyvinylpyrrolidone réticulée, l'alginate de calcium, le charbon actif, les particules de poly(chlorure de vinylidène/acrylonitrile), notamment celles commercialisées sous la dénomination générale d'« Expancel^{®} » par la société AKZO NOBEL sous les références particulières « Expancel^{®} WE» ou « DE » Expancels, et leurs mélanges.

D'une manière générale, les compositions (A) et (B) sont formulées dans un milieu cosmétiquement acceptable, qui comprend généralement de l'eau et/ou un ou plusieurs solvants organiques.

A titre de solvant organique, on peut par exemple citer les alcanols, linéaires ou ramifiés, en C₂-C₄, tels que l'éthanol et l'isopropanol ; le glycérol ; les polyols et éthers de polyols comme le 2-butoxyéthanol, le propylèneglycol, le dipropylèneglycol, le monométhyléther de propylèneglycol, le monoéthyléther et le monométhyléther du diéthylèneglycol, ainsi que les alcools aromatiques comme l'alcool benzylique ou le phénoxyéthanol, et leurs mélanges.

De tels solvants organiques peuvent être présents dans des proportions de préférence comprises entre 1 et 40 % en poids par rapport au poids total de chaque composition dans laquelle ils sont contenus, et plus préférentiellement entre 5 et 30 % en poids.

De préférence, les compositions (A) et (B) comprennent de l'eau. De manière plus préférée, chacune des compositions (A) et (B) comprend au moins 5 % en poids d'eau, de préférence au moins 10 % en poids d'eau, et mieux encore au moins 20 % en poids d'eau, par rapport à son poids total.

Les compositions (A) et/ou (B) selon la présente invention peuvent également comprendre un ou plusieurs adjuvants, choisis parmi ceux utilisés classiquement dans les compositions pour la coloration et/ou la décoloration des fibres kératiniques tels que des polymères conditionneurs en particulier cationiques ; des agents épaississants minéraux, et en particulier des charges telles que des argiles, le talc ; des agents épaississants organiques, et en particulier les épaississants associatifs polymèriques anioniques, cationiques, et non ioniques; des agents antioxydants ; des agents de pénétration ; des agents séquestrants différents de ceux de formule (I) ci-avant ; des parfums ; des agents dispersants ; des agents filmogènes ; des céramides ; des agents conservateurs ; des agents opacifiants.

Les adjuvants ci dessus peuvent être en général présents en quantité comprise pour chacun d'eux entre 0,01 et 20 % en poids par rapport au poids de chaque composition.

Selon un mode de réalisation préféré, la composition (A) se présente sous la forme d'une émulsion huile-dans-eau (dite émulsion directe), ou d'une émulsion eau-dans-huile (dite émulsion inverse).

La présente invention a également pour objet un procédé de coloration et/ou de décoloration des fibres kératiniques, comprenant l'application sur lesdites fibres de l'agent tel que décrit ci-avant.

Selon l'invention, l'agent appliqué sur les fibres kératiniques résulte du mélange des compositions (A) et (B), ce mélange étant réalisé soit avant application sur les fibres kératiniques (préparation extemporanée) soit directement sur les fibres kératiniques (application successive sur les fibres des compositions (A) et (B) sans rinçage intermédiaire).

Ainsi, selon une première variante du procédé selon l'invention, on applique sur les fibres kératiniques, sèches ou humides, successivement et sans rinçage intermédiaire, les compositions (A), puis (B).

Selon une deuxième variante du procédé selon l'invention, on applique l'agent selon l'invention sur les fibres kératiniques, sèches ou humides, en mélangeant de manière extemporanée les compositions (A) et (B), puis en appliquant ce mélange sur lesdites fibres kératiniques.

Dans ce cas, alors la durée entre le mélange des compositions (A) et (B) et l'application du mélange sur les cheveux n'excède pas de préférence 30 minutes, de préférence 10 minutes, de manière encore préférée 5 minutes.

Indépendamment de la variante mise en oeuvre, le rapport pondéral de la quantité de composition (A) utilisée à la quantité de composition (B) utilisée peut varier de 0,2 à 3 et de préférence de 0,3 à 1.

En outre, indépendamment de la variante mise en oeuvre, le mélange présent sur les fibres (résultant soit du mélange extemporané des compositions (A) et (B) soit de l'application successive de celles-ci) est laissé en place pour une durée, en général, de l'ordre de 1 minute à 1 heure, de préférence de 5 minutes à 30 minutes.

La température durant le procédé est classiquement comprise entre la température ambiante (entre 15 à 25°C) et 80°C, de préférence entre la température ambiante et 60°C.

A l'issue du traitement, les fibres kératiniques sont éventuellement rincées à l'eau, subissent éventuellement un lavage avec un shampooing suivi d'un rinçage à l'eau, avant d'être séchées ou laissées à sécher.

Enfin, l'invention a également pour objet un dispositif à plusieurs compartiments ou « kit » de coloration et/ou de décoloration, constitué d'un premier compartiment renfermant une composition (A), et d'un deuxième compartiment renfermant une composition (B), les compositions (A) et (B) étant telles que décrites ci-avant.

Ce dispositif peut avantageusement être équipé d'un moyen permettant de délivrer sur les cheveux le mélange souhaité, tel que les dispositifs décrits dans le brevet FR 2586913.

Ce dispositif peut être accompagné d'une ou plusieurs compositions de lavage et/ou de conditionnement des fibres kératiniques, destiné à être appliquées avant et/ou après le traitement de coloration et/ou de décoloration selon l'invention.

Les exemples suivants servent à illustrer l'invention sans toutefois présenter un caractère limitatif.

### EXEMPLE

L'agent de coloration d'oxydation suivant a été préparé (dans les tableaux ci-dessous, les quantités sont exprimées en grammes) :

**Composition colorante (A) :**

| Composition | A1 | A2 |
|---|---|---|
| Huile de vaseline | 55 | 55 |
| Octyl dodécanol | 10 | 10 |
| Hectorite modifiée distéaryl diméthyl ammonium | 1,5 | 1,5 |
| Carbonate de propylène | 0,5 | 0,5 |
| Alcool oléique 10 OE | 5 | 5 |
| Propylène glycol | 2 | 2 |
| Ethanol | 2,5 | 2,5 |
| Hexylène glycol | 1 | 1 |
| Dipropylène glycol | 1 | 1 |
| Monoéthanolamine | 4,5 | 4,5 |
| POE/POP/POE (Poloxamer 184) | 9 | 9 |
| Acide ascorbique | 0,25 | 0,25 |
| Solution aqueuse à 40% d'acide diéthylene triamine pentacétique, sel pentasodique | - | 1,0 |
| Paraphénylènediamine | 0,03 | 0,03 |
| Résorcinol | 0,04 | 0,04 |
| 1-hydroxy-3-aminobenzène | 0,002 | 0,002 |
| 1-beta-hydroxyéthyloxy-2,4-diamino-benzène dichlorhydrate | 0,0003 | 0,0003 |
| Sulfate de N,N-bis(2-hydroxyethyl)-p-phenylèndiamine, 1H2O | 0,006 | 0,006 |
| Eau | Qsp 100 | Qsp 100 |

La composition A2 correspond à une composition (A) conforme à la présente invention, tandis que la composition A1 est une composition comparative ne contenant pas de composé de formule (I).

**Composition oxydante (B) :**

| Composition | B |
|---|---|
| Solution aqueuse à 50% en poids de peroxyde d'hydrogène | 12 |
| Stannate de sodium | 0,04 |
| Pyrophosphate de tétrasodium | 0,03 |
| Huile de vaseline | 20 |
| Solution aqueuse à 40% en poids de polycondensat tétraméthyl hexaméthylènediamine / dichloro 1,3-propylène ou Hexadimethrine chloride | 0,1 |
| Solution aqueuse à 40% en poids de chlorure de poly diméthyl diallyl ammonium ou Polyquaternium-6, non stabilisé | 0,2 |
| Glycérine | 0,5 |
| Alcool cétéarylique | 8 |
| Alcool cétylstéarylique oxyéthyléné (33 OE) | 3 |
| Amide d'acides de colza oxyéthyléné (4 OE) | 1,2 |
| Vitamine E : DL-α-tocophérol | 0,1 |
| Acide phosphorique | Qs pH 2,2 |
| Eau | Qsp 100 |

Les compositions décrites ci-dessus ont été mélangées au moment de l'emploi, de la manière suivante :
- 10 g de la composition colorante A1 a été mélangé à 10 g de la composition oxydante B d'une part, et
- 10 g de la composition colorante A2 a été mélangé à 10 g de la composition oxydante B d'autre part.

Le mélange résultant des compositions A2 et B ne présente pas de gonflement intempestif dans le temps, contrairement au mélange des compositions A1 et B.

Appliqué sur des cheveux, le mélange résultant des compositions A2 et B conduit en outre à des résultats tinctoriaux très homogènes.

## Revendications

1. Agent de coloration et/ou de décoloration des fibres kératiniques, constitué par :
- une première composition (A) comprenant un ou plusieurs agents alcalinisants, et
- une seconde composition (B) comprenant un ou plusieurs agents oxydants,
l'une au moins des deux compositions (A) et (B) comprenant un ou plusieurs corps gras ne contenant pas de fonction acide carboxylique, la quantité totale desdits corps gras dans le mélange des compositions (A) et (B) représentant au moins 20% en poids, par rapport au poids total dudit mélange, et
l'une au moins des deux compositions (A) et (B) comprenant un ou plusieurs agents séquestrants de formule (I) suivante : 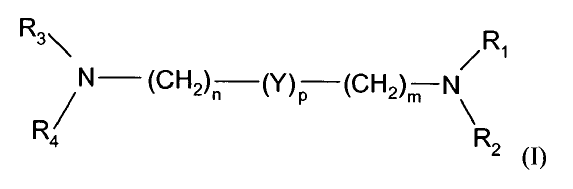 dans laquelle :
p est un nombre entier valant 0 ou 1,
n et m sont des nombres entiers valant, indépendamment l'un de l'autre, 0, 1 ou 2, la somme n + m valant au moins 1,
R₁, R₂, R₃ et R₄ représentent, indépendamment les uns des autres, un atome d'hydrogène, un groupe -CH₂CO₂M1, ou un groupe -CH(CO₂M2)(CH₂CO₂M3),
Y représente un groupe NCH₂CO₂M4,
M1 à M4 désignant, indépendamment les uns des autres, un atome d'hydrogène, un cation issu d'un métal alcalin ou d'un métal alcalino-terreux, un cation issu d'une amine organique éventuellement hydroxylée, ou un cation ammonium,
à la condition que, si p vaut 0, alors R₁ et R₃ représentent tous deux un atome d'hydrogène, et R₂ et R₄ représentent tous deux un groupe -CH(CO₂M2)(CH₂CO₂M3), et
si p vaut 1, alors n et m valent chacun au moins 1.

2. Agent selon la revendication 1, **caractérisé en ce que** l'agent alcalinisant est choisi parmi l'ammoniaque, les carbonates alcalins, les alcanolamines ainsi que leurs dérivés, les hydroxydes de sodium ou de potassium, et les composés de formule (II) suivante : 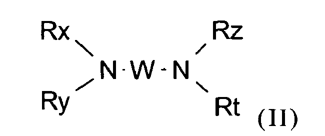 dans laquelle W est un reste alkylène en C₁-C₆ éventuellement substitué par un groupement hydroxyle ou un radical alkyle en C₁-C_{6;} Rx, Ry, Rz et Rt, identiques ou différents, représentent un atome d'hydrogène, un radical alkyle en C₁-C₆, hydroxyalkyle en C₁-C₆, ou aminoalkyle en C₁-C₆;
et est de préférence choisi parmi les alcanolamines.

3. Agent selon l'une quelconque des revendications précédentes, **caractérisé en ce que** l'agent oxydant est choisi parmi le peroxyde d'hydrogène, le peroxyde d'urée, les bromates ou ferricyanures de métaux alcalins, les sels peroxygénés comme par exemple les persulfates, les perborates et les percarbonates de métaux alcalins ou alcalino-terreux, les enzymes d'oxydo-réduction telles que les laccases, les peroxydases et les oxydoréductases à 2 électrons, éventuellement en présence de leur donneur ou cofacteur respectif; et est de préférence le peroxyde d'hydrogène.

4. Agent selon l'une quelconque des revendications précédentes, **caractérisé en ce que**, dans la formule (I), M1 à M4 sont identiques, et désignent un cation sodium ou un cation potassium.

5. Agent selon l'une quelconque des revendications précédentes, **caractérisé en ce que** l'agent séquestrant de formule (I) est choisi parmi :
- l'acide diethylènetriamine pentaacétique (DTPA) et ses sels,
- l'acide éthylènediamine disuccinique (EDDS) et ses sels, les sels de métaux alcalins de ces deux composés étant préférés.

6. Agent selon l'une quelconque des revendications précédentes, **caractérisé en ce que** la quantité totale d'agent séquestrant de formule (I) dans le mélange des deux compositions (A) et (B) va de 0,001 à 10% en poids, de préférence de 0,01 à 5% en poids, et mieux encore de 0,05 à 1% en poids, par rapport au poids total dudit mélange, ces pourcentages en poids étant exprimés par rapport à la forme acide du ou des composés de formule (I).

7. Agent selon l'une quelconque des revendications précédentes, **caractérisé en ce que** la quantité totale de corps gras ne contenant pas de fonction acide carboxylique dans le mélange des compositions (A) et (B) représente au moins 25% en poids, et de préférence au moins 30% en poids, par rapport au poids total dudit mélange.

8. Agent selon l'une quelconque des revendications précédentes, **caractérisé en ce que** le ou les corps gras ne contenant pas de fonction acide carboxylique sont choisis parmi les alcanes inférieurs en C₆-C₁₆, les huiles non siliconées d'origine minérale, végétale, animale ou synthétique, les alcools gras, les esters d'acide gras, les esters d'alcool gras, les cires non siliconées et les silicones.

9. Agent selon l'une quelconque des revendications précédentes, **caractérisé en ce que** le ou les corps gras ne contenant pas de fonction acide carboxylique sont des composés liquides à la température de 25°C et à la pression atmosphérique, et sont de préférence choisis parmi l'huile de vaseline, les polydécènes, les esters d'acides gras et/ou d'alcools gras liquides, et leurs mélanges, et plus préférentiellement l'huile de vaseline.

10. Agent selon l'une quelconque des revendications précédentes, **caractérisé en ce que** la composition (A) comprend en outre un ou plusieurs colorants d'oxydation, choisis parmi les bases d'oxydation, éventuellement combinée(s) à un ou plusieurs coupleurs, et/ou un ou plusieurs colorants directs.

11. Agent selon l'une quelconque des revendications 1 à 9, **caractérisé en ce que** les compositions (A) et (B) ne comprennent pas de colorant direct ni de colorant d'oxydation (bases et coupleurs) ou bien, s'ils sont présents, leur teneur totale ne dépasse pas 0,005% en poids par rapport au poids total de chaque composition.

12. Agent selon l'une quelconque des revendications précédentes, **caractérisé en ce que** la composition (A) se présente sous la forme d'une émulsion huile-dans-eau, ou d'une émulsion eau-dans-huile.

13. Procédé de coloration et/ou de décoloration des fibres kératiniques, comprenant l'application sur lesdites fibres, sèches ou humides, successivement et sans rinçage intermédiaire, des compositions (A) puis (B) telles que définies dans l'une quelconque des revendications précédentes.

14. Procédé de coloration et/ou de décoloration des fibres kératiniques, comprenant l'application de l'agent tel que défini dans l'une quelconque des revendications 1 à 12 sur lesdites fibres, sèches ou humides, en mélangeant de manière extemporanée les compositions (A) et (B), puis en appliquant ce mélange sur lesdites fibres.

15. Dispositif à plusieurs compartiments ou « kit » de coloration et/ou de décoloration, constitué d'un premier compartiment renfermant une composition (A), et d'un deuxième compartiment renfermant une composition (B), les compositions (A) et (B) étant telles que définies dans l'une quelconque des revendications 1 à 12.

## Claims

1. Agent for dyeing and/or bleaching keratin fibres, formed by:
- a first composition (A) comprising one or more basifying agents, and
- a second composition (B) comprising one or more oxidizing agents,
at least one of the two compositions (A) and (B) comprising one or more fatty substances not containing any carboxylic acid functions, the total amount of the said fatty substances in the mixture of compositions (A) and (B) representing at least 20% by weight relative to the total weight of the said mixture, and
at least one of the two compositions (A) and (B) comprising one or more sequestrants of formula (I) below: 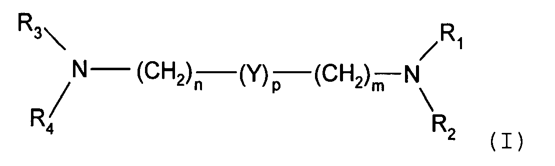 in which:
p is an integer equal to 0 or 1,
n and m are integers, independently of each other, equal to 0, 1 or 2, the sum n + m being equal to at least 1,
R₁, R₂, R₃ and R₄ represent, independently of each other, a hydrogen atom, a group -CH₂CO₂M1 or a group -CH (CO₂M2) (CH₂CO₂M3),
Y represents a group NCH₂CO₂M4,
M1 to M4 denoting, independently of each other, a hydrogen atom, a cation derived from an alkali metal or an alkaline-earth metal, a cation derived from an optionally hydroxylated organic amine, or an ammonium cation,
on condition that if p is 0, then R, and R₃ both represent a hydrogen atom, and R₂ and R₄ both represent a group -CH(CO₂M2) (CH₂CO₂M3), and
if p is 1, then n and m are each at least 1.

2. Agent according to Claim 1, **characterized in that** the basifying agent is chosen from aqueous ammonia, alkali metal carbonates, alkanolamines and derivatives thereof, sodium hydroxide, potassium hydroxide and the compounds of formula (II) below: 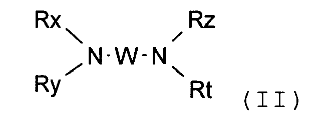 in which W is a C₁-C₆ alkylene residue optionally substituted with a hydroxyl group or a C₁-C₆ alkyl radical; Rx, Ry, Rz and Rt, which may be identical or different, represent a hydrogen atom or a C₁-C₆ alkyl, C₁-C₆ hydroxyalkyl or C₁-C₆ aminoalkyl radical;
and is preferably chosen from alkanolamines.

3. Agent according to either of the preceding claims, **characterized in that** the oxidizing agent is chosen from hydrogen peroxide, urea peroxide, alkali metal bromates or ferricyanides, peroxygenated salts, for instance alkali metal or alkaline-earth metal persulfates, perborates and percarbonates, redox enzymes such as laccases, peroxidases and 2-electron oxidoreductases, optionally in the presence of the respective donor or cofactor thereof; and is preferably hydrogen peroxide.

4. Agent according to any one of the preceding claims, **characterized in that**, in formula (I), M1 to M4 are identical, and denote a sodium cation or a potassium cation.

5. Agent according to any one of the preceding claims, **characterized in that** the sequestrant of formula (I) is chosen from:
- diethylenetriaminepentaacetic acid (DTPA) and salts thereof,
- ethylenediaminedisuccinic acid (EDDS) and salts thereof,
the alkali metal salts of these two compounds being preferred.

6. Agent according to any one of the preceding claims, **characterized in that** the total amount of sequestrant of formula (I) in the mixture of the two compositions (A) and (B) ranges from 0.001% to 10% by weight, preferably from 0.01% to 5% by weight and better still from 0.05% to 1% by weight relative to the total weight of the said mixture, these weight percentages being expressed relative to the acid form of the compound(s) of formula (I).

7. Agent according to any one of the preceding claims, **characterized in that** the total amount of fatty substances not containing any carboxylic acid functions in the mixture of compositions (A) and (B) represents at least 25% by weight and preferably at least 30% by weight relative to the total weight of the said mixture.

8. Agent according to any one of the preceding claims, **characterized in that** the fatty substance(s) not containing any carboxylic acid functions are chosen from C₆-C₁₆ lower alkanes, non-silicone oils of mineral, plant, animal or synthetic origin, fatty alcohols, fatty acid esters, fatty alcohol esters, non-silicone waxes and silicones.

9. Agent according to any one of the preceding claims, **characterized in that** the fatty substance(s) not containing any carboxylic acid functions are compounds that are liquid at a temperature of 25°C and at atmospheric pressure, and are preferably chosen from liquid petroleum jelly, polydecenes, liquid esters of fatty acids and/or of fatty alcohols, and mixtures thereof, and more preferentially liquid petroleum jelly.

10. Agent according to any one of the preceding claims, **characterized in that** composition (A) also comprises one or more oxidation dyes, chosen from oxidation bases, optionally combined with one or more couplers and/or one or more direct dyes.

11. Agent according to any one of Claims 1 to 9, **characterized in that** compositions (A) and (B) do not comprise any direct dyes or any oxidation dyes (bases and couplers), or else, if they are present, their total content does not exceed 0.005% by weight relative to the total weight of each composition.

12. Agent according to any one of the preceding claims, **characterized in that** composition (A) is in the form of an oil-in-water emulsion or of a water-in-oil emulsion.

13. Process for dyeing and/or bleaching keratin fibres, comprising the application of compositions (A) and then (B) as defined in any one of the preceding claims, successively and without intermediate rinsing, to the said wet or dry fibres.

14. Process for dyeing and/or bleaching keratin fibres, comprising the application of as defined in any one of Claims 1 to 12 to the said wet or dry fibres, the agent by extemporaneously mixing compositions (A) and (B) and in then applying this mixture to the said fibres.

15. Multi-compartment dyeing and/or bleaching device or "kit", formed from a first compartment containing a composition (A), and a second compartment containing a composition (B), compositions (A) and (B) being as defined in any one of Claims 1 to 12.

## Patentansprüche

1. Mittel zum Färben und/oder Bleichen von Keratinfasern, bestehend aus:
- einer ersten Zusammensetzung (A), die ein oder mehrere Alkalinisierungsmittel umfasst, und
- einer zweiten Zusammensetzung (B), die ein oder mehrere Oxidationsmittel umfasst,
wobei mindestens eine der beiden Zusammensetzungen (A) und (B) eine oder mehrere Fettsubstanzen, die keine Carbonsäurefunktion enthalten, umfasst, wobei die Gesamtmenge der Fettsubstanzen in der Mischung der Zusammensetzungen (A) und (B) mindestens 20 Gew.-%, bezogen auf das Gesamtgewicht der Mischung, ausmacht, und
mindestens eine der beiden Zusammensetzungen (A) und (B) ein oder mehrere Sequestriermittel der folgenden Formel (I) umfasst: 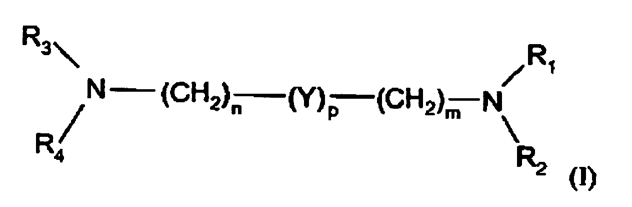 worin:
p für eine ganze Zahl mit einem Wert von 0 oder 1 steht,
n und m für ganze Zahlen stehen, die unabhängig voneinander einen Wert von 0, 1 oder 2 haben, wobei die Summe n + m mindestens 1 beträgt,
R₁, R₂, R₃ und R₄ unabhängig voneinander für ein Wasserstoffatom, eine -CH₂CO₂M1-Gruppe oder eine -CH(CO₂M2) (CH₂CO₂M3)-Gruppe stehen,
Y für eine NCH₂CO₂M4-Gruppe steht,
wobei M1 bis M4 unabhängig voneinander für ein Wasserstoffatom, ein von einem Alkalimetall oder einem Erdalkalimetall abgeleitetes Kation, ein von einem gegebenenfalls hydroxylierten organischen Amin abgeleitetes Kation oder ein Ammoniumkation stehen,
mit der Maßgabe, dass dann, wenn p gleich 0 ist, R₁ und R₃ beide für ein Wasserstoffatom stehen und R₂ und R₄ beide für eine -CH(CO₂M2)(CH₂CO₂M3)-Gruppe stehen, und
dann, wenn p gleich 1 ist, n und m jeweils mindestens gleich 1 sind.

2. Mittel nach Anspruch 1, **dadurch gekennzeichnet, dass** das Alkalinisierungsmittel unter wässrigem Ammoniak, Alkalimetallcarbonaten, Alkanolaminen sowie Derivaten davon, Natrium- oder Kaliumhydroxid und den Verbindungen der folgenden Formel (II) ausgewählt ist: 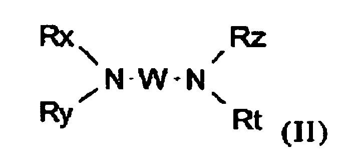 worin W für einen C₁-C₆-Alkylenrest, der gegebenenfalls durch eine Hydroxylgruppe oder einen C₁-C₆-Alkylrest substituiert ist, steht;
Rx, Ry, Rz und Rₜ gleich oder verschieden sind und für ein Wasserstoffatom, einen C₁-C₆-Alkylrest, einen C₁-C₆-Hydroxyalkylrest oder einen C₁-C₆-Aminoalkylrest stehen;
und vorzugsweise unter Alkanolaminen ausgewählt ist.

3. Mittel nach einem der vorhergehenden Ansprüche, **dadurch gekennzeichnet, dass** das Oxidationsmittel unter Wasserstoffperoxid, Harnstoffperoxid, Alkalimetallbromaten oder -ferricyaniden, Peroxysalzen wie zum Beispiel Alkalimetall- oder Erdalkalimetallpersulfaten, -perboraten und -percarbonaten, Redoxenzymen wie Laccasen, Peroxidasen und 2-Elektronen-Oxidoreduktasen, gegebenenfalls in Gegenwart des jeweiligen Donors oder Cofaktors davon, ausgewählt ist und vorzugsweise Wasserstoffperoxid ist.

4. Mittel nach einem der vorhergehenden Ansprüche, **dadurch gekennzeichnet, dass** in der Formel (I) M1 bis M4 gleich sind und für ein Natriumkation oder ein Kaliumkation stehen.

5. Mittel nach einem der vorhergehenden Ansprüche, **dadurch gekennzeichnet, dass** das Sequestriermittel der Formel (I) unter
- Diethylentriaminpentaessigsäure (DTPA) und Salzen davon,
- Ethylendiamindibernsteinsäure (EDDS) und Salzen davon
ausgewählt ist, wobei Alkalimetallsalze dieser beiden Verbindungen bevorzugt sind.

6. Mittel nach einem der vorhergehenden Ansprüche, **dadurch gekennzeichnet, dass** die Gesamtmenge an Sequestriermittel der Formel (I) in der Mischung der beiden Zusammensetzungen (A) und (B) 0,001 bis 10 Gew.-%, vorzugsweise 0,01 bis 5 Gew.-% und noch besser 0,05 bis 1 Gew.-%, bezogen auf das Gesamtgewicht der Mischung, beträgt, wobei diese Gewichtsprozentangaben sich auf die Säureform der Verbindung der Formel (I) bzw. der Verbindungen der Formel (I) beziehen.

7. Mittel nach einem der vorhergehenden Ansprüche, **dadurch gekennzeichnet, dass** die Gesamtmenge an Fettsubstanz, die keine Carbonsäurefunktion enthält, in der Mischung der Zusammensetzungen (A) und (B) mindestens 25 Gew.-% und vorzugsweise mindestens 30 Gew.-%, bezogen auf das Gesamtgewicht der Mischung, beträgt.

8. Mittel nach einem der vorhergehenden Ansprüche, **dadurch gekennzeichnet, dass** die Fettsubstanz, die keine Carbonsäurefunktion enthält, bzw. die Fettsubstanzen, die keine Carbonsäurefunktion enthalten, unter C₆-C₁₆-Niederalkanen, Nichtsilikon-ölen mineralischer, pflanzlicher, tierischer oder synthetischer Herkunft, Fettalkoholen, Fettsäureestern, Fettalkoholestern, Nichtsilikonwachsen und Silikonen ausgewählt sind.

9. Mittel nach einem der vorhergehenden Ansprüche, **dadurch gekennzeichnet, dass** die Fettsubstanz, die keine Carbonsäurefunktion enthält, bzw. die Fettsubstanzen, die keine Carbonsäurefunktion enthalten, Verbindungen sind, die bei Normaldruck bei 25°C flüssig sind, und vorzugsweise unter Vaselineöl, Polydecenen, flüssigen Fettsäure-und/oder Fettalkoholestern und Mischungen davon und weiter bevorzugt Vaselineöl ausgewählt sind.

10. Mittel nach einem der vorhergehenden Ansprüche, **dadurch gekennzeichnet, dass** die Zusammensetzung (A) außerdem ein oder mehrere Oxidationsfarbstoffe, die unter Oxidationsbasen, gegebenenfalls in Kombination mit einem oder mehreren Kupplern und/oder einem oder mehreren Direktfarbstoffen ausgewählt sind, umfasst.

11. Mittel nach einem der Ansprüche 1 bis 9, **dadurch gekennzeichnet, dass** die Zusammensetzungen (A) und (B) keine Direktfarbstoffe oder Oxidationsfarbstoffe (Basen und Kuppler) umfassen oder dann, wenn sie vorhanden sind, ihr Gesamtgehalt höchstens 0,005 Ges.-%, bezogen auf das Gesamtgewicht jeder Zusammensetzung, beträgt.

12. Mittel nach einem der vorhergehenden Ansprüche, **dadurch gekennzeichnet, dass** die Zusammensetzung (A) in Form einer Öl-in-Wasser-Emulsion oder einer Wasser-in-Öl-Emulsion vorliegt.

13. Verfahren zum Färben und/oder Bleichen von Keratinfasern, bei dem man auf die trockenen oder feuchten Fasern nacheinander und ohne Zwischenspülung die Zusammensetzungen (A) und dann (B) gemäß einem der vorhergehenden Ansprüche aufbringt.

14. Verfahren zum Färben und/oder Bleichen von Keratinfasern, bei dem man auf die trockenen oder feuchten Fasern das Mittel gemäß einem der Ansprüche 1 bis 12 aufbringt, indem man die Zusammensetzungen (A) und (B) unmittelbar zuvor mischt und diese Mischung dann auf die Fasern aufbringt.

15. Vorrichtung mit mehreren Kompartimenten oder "Kit" zum Färben und/oder Bleichen, bestehend aus einem ersten Kompartiment, das eine Zusammensetzung (A) enthält, und einem zweiten Kompartiment, das eine Zusammensetzung (B) enthält, wobei die Zusammensetzungen (A) und (B) wie in einem der Ansprüche 1 bis 12 definiert sind.
